# EUROPEAN PATENT SPECIFICATION

(11) **EP 3 260 071 B1**
(45) Date of publication and mention of the grant of the patent: **19.02.2020**
(21) Application number: 16779986.5
(22) Date of filing: 08.04.2016
(51) Int. Cl.: A61B 18/12, A61B 17/29

(54) **MEDICAL TREATMENT DEVICE, CONTROL DEVICE, AND MEDICAL TREATMENT TOOL**
VORRICHTUNG ZUR MEDIZINISCHEN BEHANDLUNG, STEUERUNGSVORRICHTUNG UND WERKZEUG ZUR MEDIZINISCHEN BEHANDLUNG
DISPOSITIF DE TRAITEMENT MÉDICAL, DISPOSITIF DE COMMANDE, ET INSTRUMENT MÉDICAL

(30) Priority: 13.04.2015 JP 2015082021
(43) Date of publication of application: 27.12.2017
(73) Proprietor: Olympus Corporation, Hachioji-shi, Tokyo 192-8507 (JP)
(72) Inventor: HIRAI, Yuji, Hachioji-shi, Tokyo 192-8507 (JP); KITAYAMA, Tadashi, Hachioji-shi, Tokyo 192-8507 (JP)
(74) Representative: Schicker, Silvia
(86) International application number: PCT/JP2016/061587
(87) International publication number: WO 2016/167195

(56) References cited:
- WO-A1-99/66850
- JP-A- 2010 538 796
- US-A1- 2013 237 982
- US-A1- 2013 267 975
- US-A1- 2013 274 729

## Description

### Technical Field

The present invention relates to a medical treatment device capable of being caused to function in a plurality of modes, and also relates to a control device and a medical treatment tool that are provided in the medical treatment device.

### Background Art

JP 2006-340839 A discloses a medical treatment device that includes a housing, a handle capable of opening and closing with respect to the housing, and an end effector with which treatment is given. In the medical treatment device, the handle is positioned near the base of a grip of the housing. Furthermore, a surface of a head of the housing (a portion near the head of the housing) is provided with a first operation button that is an operation member (a first input device), and a second operation button that is a selection member (a second input device). Inputting operation with the first operation button (pressing the first operation button) turns the first operation button into an ON state. This transmits ultrasonic vibration to the end effector (a treatment probe). With the second operation button, operation for switching the supply of high frequency energy to the end effector, or switching the intensity of electrical energy to be transmitted to an ultrasonic vibrator is performed. Thus, for example, when the supply of high frequency energy is switched with the second operation button and the first operation button is in the ON state, the medical treatment device can function in a first mode or a second mode; a first mode in which only ultrasonic vibration is transmitted to the end effector, and a second mode in which ultrasonic vibration is transmitted to the end effector and high frequency energy is also supplied to the end effector. When the first operation button (the operation member) is in the ON state, the first mode and the second mode are switched depending on the operating state input with the second operation button (the selection member).

US 2013/237982 A1 discloses an electrosurgical apparatus including a housing, provided with a plurality of buttons, and a slider. The apparatus further comprises a transformer which is connected to a generator independent from the apparatus. The slider is configured to operate a switch such that, in a condition where a first terminal and a second terminal are connected to each other, electric energy can be supplied only from the transformer to a conductive tube, while it cannot be supplied from the generator to the conductive tube. On the other hand, in a condition where the first terminal and a third terminal are connected to each other, electric energy can be supplied only from the generator to the conductive tube, while it cannot be supplied from the transformer to the conductive tube.

US 2013/267975 A1 discloses a treatment device comprising a housing segment having a plurality of buttons. When an operation input is provided via a first button among the plurality of buttons, a generator executes a predefined algorithm. On the other hand, as an operation input is provided via a second button among the plurality of buttons, the generator executes an algorithm which can be the same as or different from the one that is executed when the operation input is provided via the first button.

US 2013/274729 A1 discloses an electrosurgical system wherein a state of energy which is output from a generator to a forceps is configured to vary between a condition where a first switch is closed and a condition where a second switch is closed.

JP 2010-538796 A discloses an electrosurgical instrument in which, starting from a state where energy output from a RF source to a treatment device is provided in a first mode, the energy output mode may be switched to a second mode based on a change in an operation parameter (e.g., a jaw closing degree, a jaw closing rate, impedance of a tissue, a time interval, or an axis motion of a reciprocating member).

WO 99/66850 A1 discloses an electrosurgical treatment device capable of applying a high-frequency current to a tissue that is sandwiched between jaws.

### Summary of Invention

When a medical treatment tool is provided with a handle near the base of the grip of the housing as described in JP 2006-340839 A, an operator holds using the operator's thumb put on the handle and the operator's middle finger, ring finger, and little finger put on the grip. Then, the operator rotates a rotating knob around the longitudinal axis and rotates the end effector around the longitudinal axis with the index finger while inputting operation with the operation member provided at a portion near the head of the housing using the index finger. When the housing is provided with the selection member in addition to the operation member at the portion near the head of the housing in the configuration described above, the operator needs to also use the index finger to input operation with the selection member. This reduces the operability of the operation member and selection member.

The present invention has been made in view of the problems described above, and its object is to provide a medical treatment device that switches the modes in which the medical treatment device functions in accordance with the operating state input with a selection member, and ensures the operability for operating the operation member and the selection member, and also to provide a control device and a medical treatment tool that are provided in the medical treatment device.

To solve the above mentioned problems, according to one aspect of the invention, a medical treatment tool includes the features of claim 1. A medical treatment device for use in treatment of an object to be treated may include: a housing which includes a housing body extending along a longitudinal axis, and which has a base and a head; a handle which is openable and closable with respect to the housing; an end effector which is placed nearer to a head side than the housing, and which treats the object to be treated; an operation member which is placed at a portion near the head of the housing, and with which operation for causing the medical treatment device to function is input; a selection member which is placed nearer to the base of the housing than the operation member, and whose operating state is switched; and a control unit which is capable of causing the medical treatment device to function in a plurality of modes, the control unit being configured to detect the operating state of the selection member, and configured to select one of the modes as a mode in which the medical treatment device is caused to function based on a detection result of the operating state.

A control device to which a medical treatment tool is connected, the medical treatment tool, which is provided in a medical treatment device, may include: a housing which includes a housing body extending along a longitudinal axis and which has a base and a head; a handle which is openable and closable with respect to the housing; an end effector which is placed nearer to a head side than the housing and which treats an object to be treated; an operation member which is placed at a portion near the head of the housing; and a selection member which is placed nearer to the base of the housing than the operation member and whose operating state is switched, the control device including: a first detection signal line which transmits a first detection signal indicating an operating state of the operation member, with which operation for causing the medical treatment device to function is input; a second detection signal line which transmits a second detection signal indicating the operating state of the selection member; and a control unit to which the first detection signal and the second detection signal are transmitted, the control unit being capable of causing the medical treatment device to function in a plurality of modes, the control unit being configured to select one of the modes as a mode in which the medical treatment device is caused to function based on the second detection signal.

A medical treatment tool connected to a control device including a control unit, the control unit enabling a medical treatment device to function in a plurality of modes, may include: a housing which includes a housing body extending along a longitudinal axis, and which has a base and a head; a handle which is openable and closable with respect to the housing; an end effector which is placed nearer to a head side than the housing, and which treats an object to be treated; an operation member which is placed at a portion near the head of the housing, and with which operation for causing the medical treatment device to function is input; a selection member which is placed nearer to the base of the housing than the operation member, and whose operating state is switched; a first detection signal line which transmits a first detection signal indicating an operating state of the operation member; and a second detection signal line which transmits a second detection signal indicating the operating state of the selection member, and which transmits the second detection signal to the control unit so as to cause the control unit to select one of the modes as a mode in which the medical treatment device is caused to function based on the second detection signal.

### Brief Description of Drawings

Fig. 1 illustrates a schematic diagram of a medical treatment device according to a first embodiment.
Fig. 2 is an explanatory schematic diagram of an electrical connection between the medical treatment tool and control device according to the first embodiment, and a path of supply of energy used for treatment to an end effector.
Fig. 3 is a flowchart of a process that a control unit performs in accordance with the operating state input with an operation button in the first embodiment.
Fig. 4 is a schematic diagram of a state in which the housing and handle according to the first embodiment are held.
Fig. 5 is a schematic diagram of patterns of the modes in which the medical treatment device functions in the first embodiment and its exemplary variations. The patterns are switched in accordance with the operating state input with the selection button.
Fig. 6 is an explanatory schematic diagram of a plurality of modes in which the medical treatment device according to the first exemplary variation can function.
Fig. 7 is a schematic diagram of a configuration to detect the operating states input with the operation button and the selection button in a second exemplary variation.
Fig. 8 is a schematic diagram of a configuration to detect the operating states input with the operation buttons in a third exemplary variation.
Fig. 9 is a schematic diagram of a medical treatment tool according to a fourth exemplary variation when a selection lever is in an OFF state.
Fig. 10 is a schematic diagram of the medical treatment tool according to the fourth exemplary variation when the selection lever is in an ON state.
Fig. 11 is a schematic cross-sectional view of the selection lever and structure around the selection lever according to the fourth exemplary variation.
Fig. 12 is a schematic diagram of a medical treatment tool according to a fifth exemplary variation.
Fig. 13 is a schematic diagram of the selection slider and structure around the selection slider according to the fifth exemplary variation when the selection slider is in an OFF state.
Fig. 14 is a schematic diagram of the selection slider and structure around the selection slider according to the fifth exemplary variation when the selection slider is in an ON state.
Fig. 15 is a schematic diagram of a medical treatment tool according to a sixth exemplary variation.
Fig. 16 is a schematic diagram of a medical treatment tool according to a seventh exemplary variation when a selection switch is in an OFF state.
Fig. 17 is a schematic diagram of the medical treatment tool according to the seventh exemplary variation when the selection switch is in an ON state.
Fig. 18 is a schematic diagram of a medical treatment tool according to an eighth exemplary variation when a selection switch is in an OFF state.
Fig. 19 is a schematic diagram of the medical treatment tool according to the eighth exemplary variation when the selection switch is in an ON state.
Fig. 20 is a schematic diagram of a medical treatment tool according to a ninth exemplary variation when a selection switch is in an OFF state.
Fig. 21 is a schematic diagram of the medical treatment tool according to the ninth exemplary variation when the selection switch is in an ON state.
Fig. 22 is a schematic diagram of a medical treatment tool according to a tenth exemplary variation.
Fig. 23 is a schematic diagram of a configuration to detect the operating states input with the operation button, the selection button, and the selection switch.
Fig. 24 is a schematic diagram of a medical treatment tool according to an eleventh exemplary variation.
Fig. 25 is a schematic diagram of a state in which the housing and handle according to the eleventh exemplary variation are held.
Fig. 26 is a schematic diagram of a medical treatment tool according to a twelfth exemplary variation.
Fig. 27 is a schematic diagram of a medical treatment tool according to a thirteenth exemplary variation.
Fig. 28 is a schematic diagram of a state in which the housing and handle according to the thirteenth exemplary variation are held.
Fig. 29 is a schematic diagram of a medical treatment tool according to a fourteenth exemplary variation.
Fig. 30 is a schematic diagram of a medical treatment tool according to a fifteenth exemplary variation.
Fig. 31A is a schematic diagram of a state in which an external force does not act on a lever member according to the fifteenth exemplary variation.
Fig. 31B is a schematic diagram of a state in which an external force acts on a first lever extension portion in the lever member according to the fifteenth exemplary variation.
Fig. 31C is a schematic diagram of a state in which an external force acts on a second lever extension portion in the lever member according to the fifteenth exemplary variation.
Fig. 31D is a schematic diagram of a state in which an external force acts on a lever supporting shaft in the lever member according to the fifteenth exemplary variation.

The method steps described below only represent background that is useful for understanding the present invention, but do not form part of the same. Description of Embodiments

### (First Embodiment)

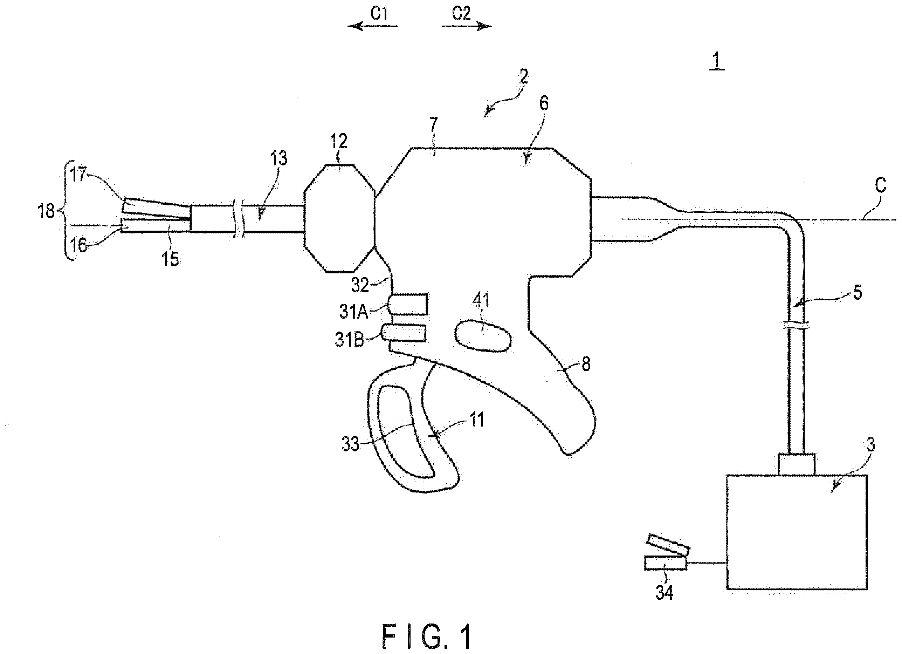

The first embodiment of the present invention will be described with reference to Figs. 1 to 4.

Fig. 1 is a diagram of a medical treatment device (medical treatment system) 1 according to the present embodiment. As illustrated in Fig. 1, the medical treatment device 1 includes a medical treatment tool (handpiece) 2. The medical treatment tool 2 has a longitudinal axis C. In this example, a first side in the direction of the longitudinal axis C is on a head side near the head of the medical treatment tool 2 (a side of an arrow C1 in Fig. 1), and the side opposite to the side near the head is on a base side near the base of the medical treatment tool 2 (a side of an arrow C2 in Fig. 1). The medical treatment tool 2 is detachably connected to a control device 3 through a cable 5. The control device 3 is an energy control device, for example, that controls the supply of energy used for treatment to (treatment energy) to the medical treatment tool 2.

The medical treatment tool 2 includes a housing 6 including a head and a base. The housing 6 includes a housing body 7 extending along the longitudinal axis C, and a grip (fixed handle) 8 extending from the housing body 7 in the direction crossing the longitudinal axis C. A first end of the cable 5 is connected to the base portion of the housing body 7. A handle (movable handle) 11 is rotatably attached to the housing 6. The handle 11 rotates around an attachment position at which the handle 11 is attached to the housing 6. This rotation allows the handle 11 to open and close with respect to the grip 8 of the housing 6 (displaces the handle 11). In the present embodiment, the handle 11 is positioned on a head side near the head of the grip 8 so that the handle 11 opens and closes with respect to the grip 8 (moves from and toward the grip 8) in roughly parallel to the longitudinal axis C.

A rotating knob 12 is coupled to the head side of the housing body 7. The rotating knob 12 is rotatable around the longitudinal axis C of the housing 6. A sheath 13 is coupled to the housing 6 while being inserted from the head side into the rotating knob 12 and housing body 7. An extension member (probe) 15 extends from the inside of the housing body 7 through the inside of the sheath 13 toward the head side. The head of the extension member 15 is provided with a first grasp unit (treatment probe) 16. The extension member 15 is inserted into and penetrates the sheath 13. The first grasp unit 16 protrudes from the head of the sheath 13 toward the head side. In the present embodiment, the extension member 15 is made of a material having high vibration transferability and thus capable of transferring ultrasonic vibration. A second grasp unit (jaw) 17 is rotatably attached to the head of the sheath 13.

The handle 11 opens or closes with respect to the grip 8. This moves a movable pipe (not illustrated) extending between the sheath 13 and the extension member 15 along the longitudinal axis C. This rotates the second grasp unit 17 so that the second grasp unit 17 opens or closes with respect to the first grasp unit 16. In the present embodiment, the first grasp unit 16 and the second grasp unit 17 form an end effector 18 that treats an object to be treated such as body tissue. The end effector 18 grasps the object to be treated between a pair of the grasp units 16 and 17 so as to treat the object to be treated.

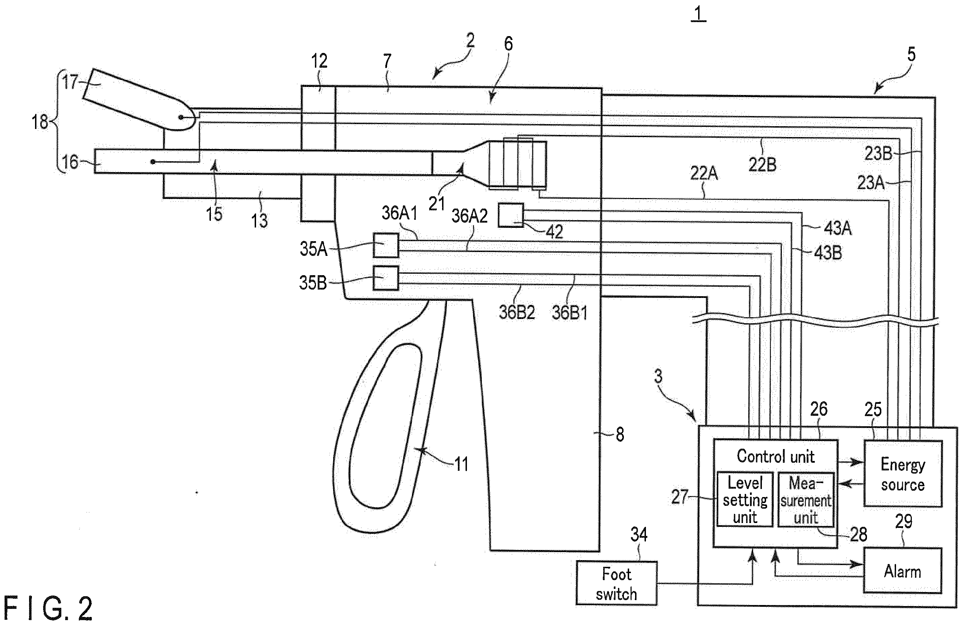

Fig. 2 is an explanatory diagram of an electrical connection between the medical treatment tool 2 and the control device 3, and a path of supply of energy used for treatment to the end effector 18. As illustrated in Figs. 1 and 2, a vibration generation unit 21 extends in the housing body 7 along the longitudinal axis C. The vibration generation unit 21 is connected to a base side near the base of the extension member 15 in the housing body 7. The vibration generation unit 21 is provided with a piezoelectric device (not illustrated) that converts electrical energy (alternating current) into ultrasonic vibration.

The control device 3 includes one or more processors such as an energy source 25 that generates energy for treatment, and a control unit 26 that controls the energy source 25. The energy source 25 includes, for example, a drive circuit (not illustrated) that converts direct current power from a battery or electricity from an outlet into electrical energy used for treatment. The control unit 26 includes, for example, a processor or an integrated circuit including a Central Processing Unit (CPU) or an application specific integrated circuit (ASIC), and a storage medium such as a memory so as to control the whole medical treatment device 1. Note that, in the control unit 26, for example, a processor can work as a level setting unit 27 configured to set a level (energy level) on each type of energy output from the energy source 25, or can work as a measurement unit 28(a measurement circuit) that can measure a predetermined parameter. The level setting unit 27 and measurement unit 28 perform some of processes that, for example, the processor performs. The control device 3 is provided also with an alarm 29 electrically connected to the control unit 26. The alarm 29 is, for example, a bell that warns by generating a sound, a lamp that warns by producing light, or a display screen that displays warning. In this example, the alarm 29 is not necessarily placed in the control device 3, and can be placed, for example, in the housing 6 of the medical treatment tool 2. Alternatively, warning can be displayed on the display screen of an endoscope (not illustrated) used together with the medical treatment device 1.

First ends of electrical lines 22A and 22B are connected to the vibration generation unit 21. The electrical lines (energy lines) 22A and 22B extend through the insides of the housing 6, cable 5, and control device 3. Second ends of the electrical lines 22A and 22B are connected to the energy source 25. The control by the control unit 26 outputs the electrical energy (alternating current power) that is to be converted into ultrasonic vibration from the energy source 25 through the electrical lines 22A and 22B, and the electrical energy output through the electrical lines 22A and 22B is supplied to the vibration generation unit 21. The supply of the electrical energy to the vibration generation unit 21 generates ultrasonic vibration. Then, the generated ultrasonic vibration is transmitted to the extension member 15. The ultrasonic vibration is transmitted from the base side to head side of the extension member 15. Subsequently, the ultrasonic vibration is transmitted to the first grasp unit 16 of the end effector 18.

First ends of energy lines 23A and 23B are also connected to the energy source 25. The energy line 23A extends through the insides of the control device 3, cable 5, and housing 6. A second end of the energy line 23A is connected to the first grasp unit 16. The energy line 23B extends through the insides of the cable 5, and housing 6. A second end of the energy line 23B is connected to the second grasp unit 17. The control by the control unit 26 outputs high frequency energy (high frequency electricity) from the energy source 25 through the energy lines 23A and 23B. Then, the high frequency energy is supplied through the energy line 23A to the first grasp unit 16 and through the energy line 23B to the second grasp unit 17. The supply of the high frequency energy to the end effector 18 (the first grasp unit 16 and second grasp unit 17) causes the first grasp unit 16 and second grasp unit 17 to function as electrodes of the high frequency energies with different electrical potentials each other.

As illustrated in Fig. 1, operation buttons 31A and 31B are attached as operation members to the housing 6. In the present embodiment, the operation buttons 31A and 31B are attached to a surface of a head 32 of the grip 8, and placed at a portion of the head side of the housing 6. The operation buttons 31A and 31B are placed on the side on which the grip 8 is positioned when the longitudinal axis C is centered, and placed nearer to the longitudinal axis C than a force application unit (handle finger-put position) 33 to which operation force is applied by operation for opening or closing the handle 11 with respect to the housing 6. With each of the operation buttons 31A and 31B, operation for causing the medical treatment device 1 to function (for example, to supply energy to the end effector 18) is input. In the present embodiment, the operation buttons 31A and 31B are momentary operation members and thus each of the operation buttons 31A and 31B is in the ON state only while the operation is input (only while the button is pressed). Thus, when the input of operation (pressing the button) is released, the operation buttons 31A and 31B is turned into an OFF state.

As illustrated in Fig. 2, the inside of the housing 6 (the inside of the grip 8) is provided with switches 35A and 35B. Each of the switches 35A and 35B is switched between an opening state and a closing state in accordance with the operating state (namely, the ON state or OFF state) input with the operation button (one of 31A and 31B) corresponding to each of the switches 35A and 35B. First ends of the detection signal lines (first detection signal lines) 36A1 and 36A2 are connected to the switch 35A. The detection signal lines 36A1 and 36A2 extend through the insides of the housing 6, cable 5, and control device 3. Second ends of the detection signal lines 36A1 and 36A2 are connected to the control unit 26. The detection signal lines 36A1 and 36A2 transmit a detection signal (first detection signal) indicating whether the switch 35A opens or closes (namely, indicating the operating state input with the operation button 31A) to the control unit 26. Then, the control unit 26 detects the operating state input with the operation button 31A (namely, whether the operation button 31A is in the ON state or the OFF state) in accordance with the detection signal transmitted through the detection signal lines 36A1 and 36A2.

Similarly, the switch 35B is connected to the control unit 26 through the detection signal lines (first detection signal lines) 36B1 and 36B2 so that the detection signal lines 36B1 and 36B2 transmit a detection signal (first detection signal) indicating whether the switch 35B opens or closes (namely, the operating state input with the operation button 31B) to the control unit 26. The control unit 26 detects the operating state input with the operation button 31B (namely, whether the operation button 31B is in the ON state or the OFF state) in accordance with the detection signal transmitted through the detection signal lines 36B1 and 36B2.

As illustrated in Fig. 1, the housing 6 is provided with a selection button 41 as a selection member. In the present embodiment, the selection button 41 is placed on the grip 8 and exposed to the outside. The selection button 41 is placed at a portion facing an end of the width direction of the housing 6 (in the direction perpendicular to the drawing paper of Fig. 1) on the external surface of the housing 6. The selection button 41 is placed nearer to the base side than the operation buttons 31A and 31B, and placed nearer to the longitudinal axis C than the force application unit 33 of the handle 11. In this example, the selection button 41 can be a momentary selection member or can be an alternate selection member. When the selection button 41 is a momentary one, the selection button 41 is in the ON state only while operation is input (only while the selection button 41 is pressed). On the other hand, when the selection button 41 is an alternate one, inputting operation input with the selection button 41 in the OFF state (pressing the selection button 41) switches the selection button 41 into the ON state. Inputting operation input with the selection button 41 in the ON state (pressing the selection button 41) switches the selection button 41 into the OFF state. Even when the input of operation (pressing the selection button 41) is released, the selection button 41 is maintained to be in the OFF state. Note that, when the selection button 41 is an alternate one, the housing 6 is provided with a state maintenance mechanism (not illustrated) that maintains the selection button 41 in the ON state and the selection button 41 in the OFF state.

As illustrated in Fig. 2, the inside of the housing 6 (the inside of the grip 8) is provided with a switch 42. The switch 42 is switched between the opening state and the closing state in accordance with the operating state input with the selection button 41 (namely, whether the selection button 41 is in the ON state or the OFF state). First ends of the detection signal lines (second detection signal lines) 43A and 43B are connected to the switch 42. The detection signal lines 43A and 43B extend through the insides of the housing 6, cable 5, and control device 3. Second ends of the detection signal lines 43A and 43B are connected to the control unit 26. The detection signal lines 43A and 43B transmit a detection signal (second detection signal) indicating whether the switch 42 opens or closes (namely, the operating state input with the selection button 41) to the control unit 26. The control unit 26 detects the operating state input with the selection button 41 (namely, whether the selection button 41 is in the ON state or the OFF state) in accordance with the detection signal transmitted through the detection signal lines 43A and 43B.

Note that an exemplary embodiment can be provided with a foot switch 34 as an operation input apparatus (third input device). In this example, the foot switch 34 is placed separately from the medical treatment tool 2 (the housing 6 and end effector 18). With the foot switch 34 working as the operation input apparatus, operation for causing the medical treatment device 1 to function is input. The input of operation with the foot switch 34 is detected by the control unit 26.

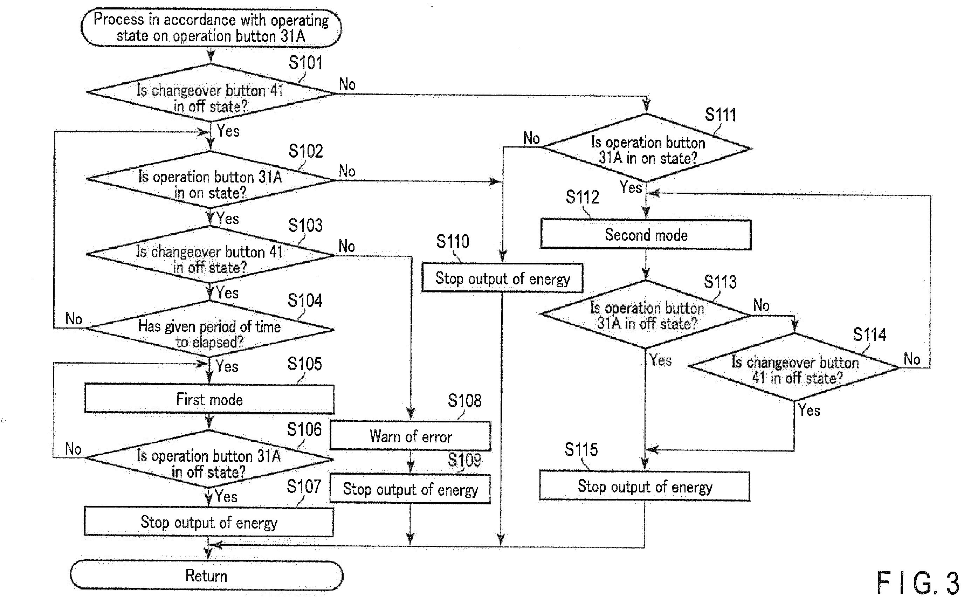

Next, the mechanisms and effects of the medical treatment tool 2, control device 3, and medical treatment device 1 according to the present embodiment will be described. Fig. 3 is a flowchart of a process that the control unit 26 (the control device 3) performs in accordance with the operating state input with the operation button (first operation button) 31A. As illustrated in Fig. 3, while the control device 3 operates, the control unit 26 detects the operating state input with the selection button 41 (namely, whether the selection button 41 is in the ON state or the OFF state) in accordance with the detection signal transmitted through the detection signal lines (second detection signal lines) 43A and 43B (step S101). When the selection button 41 is in the OFF state (step S101-Yes), the control unit 26 detects the operating state input with the operation button 31A in accordance with the detection signal transmitted through the detection signal lines (first detection signal lines) 36A1 and 36A2, and detects whether the operation button 31A is switched from the OFF state to the ON state (step S102).

When the operation button 31A is switched to the ON state (step S102-Yes), the control unit 26 detects whether the selection button 41 is maintained to be in the OFF state (step S103), and detects whether a given period of time T0 has elapsed since the operation button 31A has been switched to the ON state (step S104). In other words, the control unit 26 detects whether the selection button 41 is maintained to be in the OFF state until a given period of time T0 has elapsed since the operation button 31A has been switched to the ON state in steps S103 and S104. In this example, the control unit 26 sets the given period of time T0, for example, within a range between 0.05 and 1 seconds. Note that, when the given period of time T0 has not elapsed since the operation button 31A has been switched to the ON state in step S104 (step S104-No), the process goes back to step S102.

When the selection button 41 is maintained to be in the OFF state (step S103-Yes and S104-Yes) during the given period of time T0 after the operation button 31A is switched to the ON state, the control unit 26 causes the medical treatment device 1 to function in the first mode (step S105). This causes the medical treatment device 1 to function in the first mode when the given period of time T0 has elapsed after the operation button 31A is switched from the OFF state to the ON state while the selection button 41 is in the OFF state. In other words, the control unit 26 causes the medical treatment device 1 to function in the first mode in accordance with the fact that the operation button 31A is in the ON state when the selection button 41 is in the OFF state. In the present embodiment, by causing the medical treatment device 1 to function in the first mode, the control unit 26 supplies the electrical energy (alternating current) from the energy source 25 to the vibration generation unit 21 so as to transmit the ultrasonic vibration (the first energy) generated by the vibration generation unit 21 to the first grasp unit 16 of the end effector 18.

The control unit 26 causes the medical treatment device 1 to function in the first mode (step S105) as long as the operation button 31A is maintained to be in the ON state (step S106-No). In such a case, the medical treatment device 1 is maintained to be in the first mode regardless of the operating state input with the operation button (second operation button) 31B. In other words, unless the operation button 31A is switched from the ON state to the OFF state, the medical treatment device 1 functions in the first mode even if the operation button 31B is switched from the OFF state to the ON state. When the operation button 31A is switched from the ON state to the OFF state (step S106-Yes), the control unit 26 stops the output of energy from the energy source 25 (step S107) and thus the electrical energy is not supplied to the vibration generation unit 21. The output of energy from the energy source 25 is stopped regardless of the operating state input with the operation button 31B (namely, whether the operation button 31B is in the ON state or the OFF state). This stops the transmission of the ultrasonic vibration to the end effector 18 and stops the medical treatment device 1 from functioning.

When the selection button 41 is switched to the ON state (step S103-No) before the given period of time T0 has elapsed since the operation button 31A has been switched to the ON state in step S102, the control unit 26 causes the alarm 29 to warn an error (step S108). Meanwhile, the control unit 26 maintains the state in which the output of energy from the energy source 25 stops (step S109) . This maintains the state in which the medical treatment device 1 is stopped from functioning. For example, the operator recognizes from the error warning of the alarm 29 that operation is not properly input with the operation button 31A and the selection button 41.

When the selection button 41 is in the ON state in step S101 (step S101-No), the control unit 26 detects the operating state input with the operation button 31A in accordance with the detection signal transmitted through the detection signal lines (first detection signal lines) 36A1 and 36A2, and detects whether the operation button 31A is switched from the OFF state to the ON state (step Sill). When the operation button 31A is maintained to be in the OFF state (step S101-No or step Sill-No), the control unit 26 maintains the state in which the output of energy from the energy source 25 is stopped (step S110) regardless of the operating state input with the selection button 41 (in other words, whether the selection button 41 is in the ON state or the OFF state). This maintains the state in which the medical treatment device 1 is stopped from functioning in the process performed in accordance with the operating state input with the operation button 31A unless the operation button 31A is switched to the ON state. When the selection button 41 is in the OFF state and the operation button 31A is switched to the OFF state again before the given period of time T0 has elapsed since the operation button 31A has been switched to the ON state (step S104-No and S102-No), the control unit 26 maintains the state in which the output of energy from the energy source 25 is stopped (step S110).

When the selection button 41 is in the ON state and the operation button 31A is switched from the OFF state to the ON state (step S101-No and step Sill-Yes), the control unit 26 causes the medical treatment device 1 to function in a second mode different from the first mode (step S112). Thus, when the selection button 41 is in the ON state and the operation button 31A is switched from the OFF state to the ON state, the medical treatment device 1 functions in the second mode. In other words, the control unit 26 causes the medical treatment device 1 to function in the second mode in accordance with the fact that the selection button 41 is in the ON state and the operation button 31A is in the ON state. In the present embodiment, by causing the medical treatment device 1 to function in the second mode, the control unit 26 supplies the electrical energy (alternating current) from the energy source 25 to the vibration generation unit 21 and transmits the ultrasonic vibration (first energy) generated by the vibration generation unit 21 to the first grasp unit 16 of the end effector 18, and transmits the high frequency energy (second energy) different from the ultrasonic vibration to the end effector 18.

As long as the operation button 31A is maintained to be in the ON state (step S113-No) and the selection button 41 is maintained to be in the ON state (step S114-No), the control unit 26 causes the medical treatment device 1 to function in the second mode (step S112). In such a case, the medical treatment device 1 is maintained to be in the second mode regardless of the operating state of the operation button (second operation button) 31B. In other words, as long as the operation button 31A is not switched from the ON state to the OFF state and the selection button 41 is not switched from the ON state to the OFF state, the medical treatment device 1 functions in the second mode even if the operation button 31B is switched from the OFF state to the ON state.

When the operation button 31A is switched from the ON state to the OFF state (step S113-Yes), or when the selection button 41 is switched from the ON state to the OFF state (step S114-Yes), the control unit 26 stops the output of energy from the energy source 25 (step S115). This stops the supply of the electrical energy to the vibration generation unit 21 and thus stops the transmission of the ultrasonic vibration to the end effector 18. This also stops the supply of the high frequency energy to the end effector 18. In such a case, the output of energy from the energy source 25 is stopped regardless of the operating state input with the operation button 31B (in other words, whether the operation button 31B is in the ON state or the OFF state). This stops the supply of energy to the end effector 18, and stops the medical treatment device 1 from functioning.

A process that the control unit 26 performs in accordance with the operating state input with the operation button (second operation button) 31B is performed in a similar manner to the process performed in accordance with the operating state on the operation button (first operation button) 31A (see Fig. 3). In other words, when the selection button 41 is in the OFF state and the operation button 31B is switched from the OFF state to the ON state, the control unit 26 detects whether the selection button 41 is maintained to be in the OFF state until the given period of time T0 has elapsed since the operation button 31B has been switched to the ON state. When the selection button 41 is maintained to be in the OFF state until the given period of time T0 has elapsed since the operation button 31B has been switched to the ON state, the control unit 26 causes the medical treatment device 1 to function in a third mode different from the first mode and the second mode. In other words, the control unit 26 causes the medical treatment device 1 to function in the third mode in accordance with the fact the selection button 41 is in the OFF state and the operation button 31B is in the ON state. In the present embodiment, by causing the medical treatment device 1 to function in the third mode, the control unit 26 supplies the high frequency energy to the end effector 18. In this example, the high frequency energy is supplied in an appropriate amount, for an appropriate length of time, at an appropriate frequency for solidification.

As long as the operation button 31B is in the ON state when the medical treatment device 1 functions in the third mode, the control unit 26 maintains the medical treatment device 1 in the third mode regardless of the operating state input with the operation button 31A. When the operation button 31B is switched to the OFF state, the control unit 26 stops the output of energy from the energy source 25 and stops the medical treatment device 1 from functioning regardless of the operation input with the operation button 31A (in other words, whether the operation button 31A is in the ON state or the OFF state). When the selection button 41 is in the OFF state and the operation button 31B is switched to the ON state and the selection button 41 is switched from the OFF state to the ON state before the given period of time T0 has elapsed since the operation button 31B has been switched to the ON state, the control unit 26 warns an error and maintains the state in which the medical treatment device 1 is stopped from functioning.

When the selection button 41 is in the ON state and the operation button 31B is switched from the OFF state to the ON state, the control unit 26 causes the medical treatment device 1 to function in a fourth mode different from the first to third modes. In other words, the control unit 26 causes the medical treatment device 1 to function in the fourth mode in accordance with the fact that the selection button 41 is in the ON state and the operation button 31B is in the ON state. In the present embodiment, by causing the medical treatment device 1 to function in the fourth mode, the control unit 26 supplies the same type of high frequency energy as the high frequency energy supplied in the third mode to the end effector 18 in a state in which the high frequency energy is supplied while at least one of the amount of the supplied high frequency energy, the length of time to supply the high frequency energy, and the frequency at which the high frequency energy is supplied is different from that in the third mode. At that time, the high frequency energy is supplied in an appropriate amount, for an appropriate length of time, at an appropriate frequency for sealing of a blood vessel.

When the medical treatment device 1 functions in the fourth mode, the control unit 26 maintains the medical treatment device 1 in the fourth mode regardless of the operating state input with the operation button 31A as long as the operation button 31B is maintained to be in the ON state and the selection button 41 is maintained to be in the ON state. When the operation button 31B is switched to the OFF state, or the selection button 41 is switched to OFF state, the control unit 26 stops the output of energy from the energy source 25 and stops the medical treatment device 1 from functioning regardless of the operating state input with the operation button 31A (in other words, whether the operation button 31A is the ON state or the OFF state).

As described above, when the operation button 31A is in the ON state, the detection signal (the first detection signal) indicating the operating state input with the operation button 31A is transmitted through the detection signal lines 36A1 and 36A2 to the control unit 26. This enables the control unit 26 to cause the medical treatment device 1 to function in a plurality of modes (in the first mode or the second mode). When the operation button 31A is the ON state, the detection signal (the second detection signal) indicating the operating state input with the selection button 41 (whether the selection button 41 is in the ON state or the OFF state) is transmitted through the detection signal lines 43A and 43B to the control unit 26. Then, the control unit 26 selects (determines) a mode in which the medical treatment device 1 functions (the first mode or the second mode) from the modes in accordance with the transmitted detection signal (the second detection signal). Similarly, when the operation button 31B is in the ON state, the detection signal (the first detection signal) indicating the operating state input with the operation button 31B is transmitted through the detection signal lines 36B1 and 36B2 to the control unit 26. This enables the control unit 26 to cause the medical treatment device 1 to function in a plurality of modes (the third mode or the fourth mode). When the operation button 31B is the ON state, the detection signal (the second detection signal) indicating the operating state input with the selection button 41 (whether the selection button 41 is in the ON state or the OFF state) is transmitted through the detection signal lines 43A and 43B to the control unit 26. Then, the control unit 26 selects (determines) a mode in which the medical treatment device 1 functions (the third mode or the fourth mode) from the modes in accordance with the transmitted detection signal (the second detection signal) .

Thus, in the present embodiment, only providing a selection button (selection member) 41 enables the control unit 26 to cause the medical treatment device 1 to function in a plurality of modes when each of the operation buttons (the operation members) 31A and 31B is in the ON state by performing the process in accordance with the operating state input with the operation button 31A illustrated in Fig. 3, and the process in a similar manner to the process illustrated in Fig. 3 in accordance with the operating state input with the operation button 31B. This enables the medical treatment device 1 to function in many modes even when the number of the operation buttons 31A and 31B is reduced.

In the detection of the operating states input with the operation buttons 31A and 31B, and the selection button 41, for example, when the selection button 41 is switched from the OFF state to the ON state before the given period of time T0 has elapsed since the operation button (31A or 31B) has been switched to the ON state, it is sometimes difficult to determine whether the operation button (31A or 31B) and the selection button 41 are simultaneously in the ON state. In the present embodiment, the process in accordance with the operating state input with the operation button 31A illustrated in Fig. 3, and the process in a similar manner to the process illustrated in Fig. 3 in accordance with the operating state input with the operation button 31B are processed. By performing the processes, the control unit 26 warns an error when it is difficult to determine whether the operation button (31A or 31B) and the selection button 41 are simultaneously in the ON state. This warning effectively prevents the medical treatment device 1 from functioning in a mode that the operator does not intend.

To treat an object to be treated using the medical treatment device 1, the operator holds the housing 6 and the handle 11 and inserts the head of the sheath 13 and the end effector 18, for example, into an abdominal cavity. The operator opens or closes the handle 11 with respect to the grip 8 while inserting the sheath 13 and the end effector 18 in a body cavity in order to open or close the grasp units 16 and 17, rotate the rotating knob 12 around the longitudinal axis C in order to adjust the angle of the position of the end effector 18 around the longitudinal axis C.

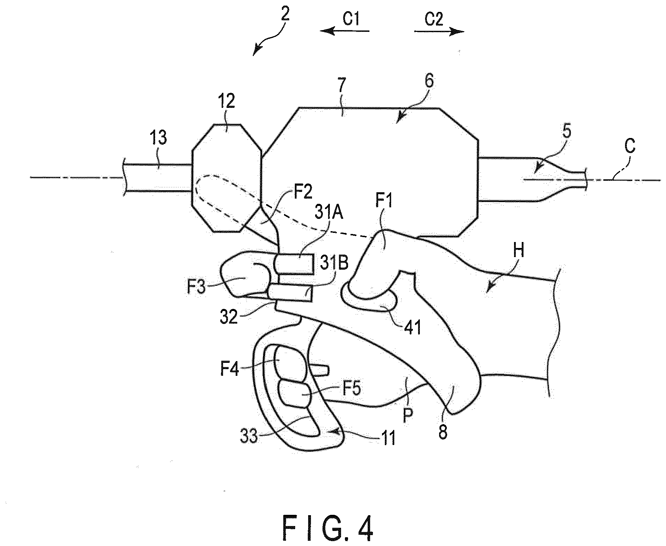

Fig. 4 is a diagram of a state in which the operator holds the housing 6 and the handle 11. As illustrated in Fig. 4, for example, when the operator holds the housing 6 and the handle 11 with the operator's right hand H, the operator puts the grip 8 of the housing 6 between the operator's thumb F1 and palm P. The operator puts the operator's ring finger F4 and little finger F5 (or middle finger F3, ring finger F4, and little finger F5 depending on the operator) on the force application unit 33 of the handle 11 so as to apply operation force on the handle 11 using the operator's ring finger F4 and little finger F5 (or middle finger F3, ring finger F4, and little finger F5 depending on the operator) in order to close the handle 11 with respect to the grip 8. The operator uses the operator's index finger F2 or middle finger F3 to rotate the rotating knob 12 and input operation with each of the operation buttons 31A and 31B (press each of the operation buttons 31A and 31B).

In the present embodiment, the selection button (selection member) 41 is placed nearer to the base side of the housing 6 than the operation buttons 31A and 31B on the outer surface of the housing 6. This placement enables the operator to input operation with the selection button 41 (to press the selection button 41) using the thumb F1 while grasping the grip 8 between the thumb F1 and the palm P. Thus, the operator can switch the selection button 41 between ON state and OFF state using the thumb F1. Thus, the operator does not use the operator's index finger F2, middle finger F3, ring finger F4, and little finger F5, which are used for at least one of operations for opening and closing the handle 11, rotating the rotating knob 12, and inputting operation with the operation buttons 31A and 31B, in order to input operation with the selection button 41. Thus, the operator does not need to change the positions and postures of the operator's index finger F2, middle finger F3, ring finger F4 and little finger F5 in order to switch the selection button 41 between the ON state and the OFF state in treatment, and does not need also to hold the housing 6 and the handle 11 again with the operator's right hand H. This secures the operability for opening and closing the handle 11, rotating the rotating knob 12, and inputting operation with the operation buttons 31A and 31B, and secures also the operability for inputting operation with the selection button 41.

As described above, the present embodiment can provide the medical treatment device (1) that switches the modes in which the medical treatment device (1) functions in accordance with the operating state input with the selection member (41), and secures the operability of the operation members (31A and 31B), and the selection member (41) .

### (Exemplary Variation)

Note that, when the operation button 31A is in the ON state in the first embodiment, the medical treatment device 1 can function in the mode in which ultrasonic vibration is transmitted to the end effector 18 (the first mode) and in the mode in which ultrasonic vibration is transmitted and the high frequency energy is also supplied to the end effector 18 (the second mode) in accordance with the operating state input with the selection button 41. However, the mode is not limited to the modes according to the first embodiment. Similarly, when the operation button 31B is in the ON state, the medical treatment device 1 can function in the mode in which high frequency energy is supplied to the end effector 18 in an amount appropriate for solidification (the third mode) and in the mode in which high frequency energy is supplied to the end effector 18 in an amount appropriate for sealing of a blood vessel (the fourth mode) in accordance with the operating state input with the selection button 41. However, the mode is not limited to the modes according to the first embodiment.

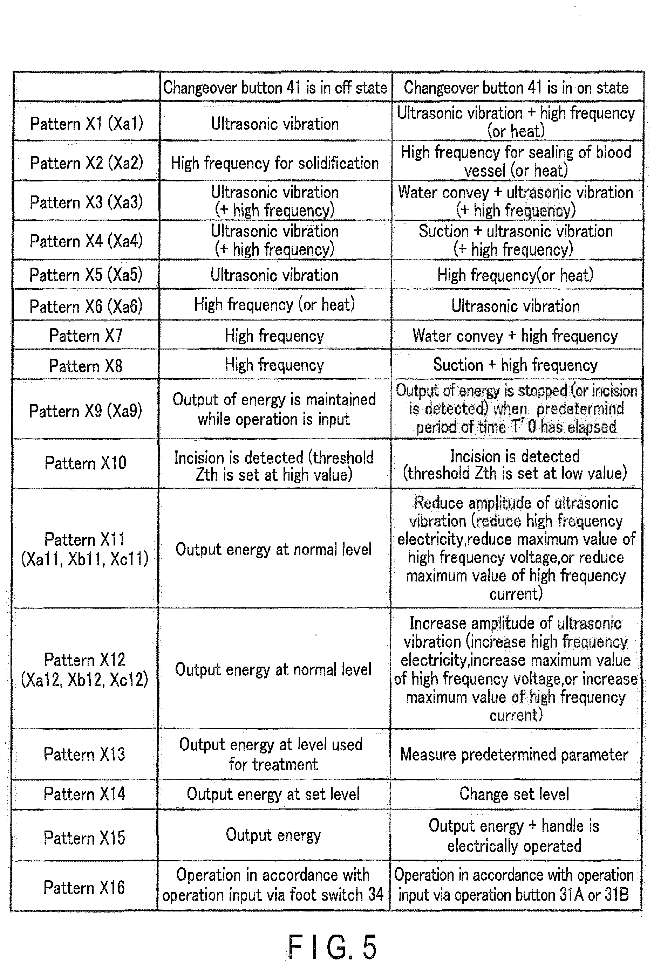

Fig. 5 is a diagram (table) of patterns of switching of the modes in which the medical treatment device 1 functions in accordance with the operating state input with the selection button 41. When the operation button 31A is in the ON state in the first embodiment, the medical treatment device 1 functions in a pattern X1 illustrated in Fig. 5 in accordance with the operating state input with the selection button 41. When the operation button 31B is in the ON state, the medical treatment device 1 functions in a pattern X2 illustrated in Fig. 5 in accordance with the operating state input with the selection button 41. However, in an exemplary variation, the medical treatment device 1 functions in the pattern X2 when the operation button 31A is in the ON state and the medical treatment device 1 functions in the pattern X1 when the operation button 31B is in the ON state. In other words, the control unit 26 causes the medical treatment device 1 to function, for example, in one of patterns X1 to X16, Xa1 to Xa6, Xa9, Xa11 to Xc11, and Xa12 to Xc12 illustrated in Fig. 5 in accordance with the operating state input with the selection button 41.

The first embodiment is provided with two operation buttons 31A and 31B. However, the number of the operation buttons can be one, or three or more. In other word, at least an operation button (31A or 31B) needs being provided. When one operation button is provided, the control unit 26 causes the medical treatment device 1 to function in one of the patterns X1 to X16, Xa1 to Xa6, Xa9, Xa11 to Xc11, and Xa12 to Xc12 illustrated in Fig. 5 in accordance with the operating state input with the selection button 41.

Hereinafter, each of the patterns X1 to X16, Xa1 to Xa6, Xa9, Xa11 to Xc11, and Xa12 to Xc12 illustrated in Fig. 5 will be described. Note that the patterns X1 and X2 have already described in the first embodiment, and thus the description will be omitted. In the pattern Xa1, the medical treatment device 1 functions in a mode in which ultrasonic vibration is transmitted to the end effector 18 in accordance with the fact the selection button 41 is in the OFF state and the operation button (31A or 31B) is in the ON state. The medical treatment device 1 functions in a mode in which the ultrasonic vibration and heat are transmitted to the end effector 18 in accordance with the fact the selection button 41 is in the ON state and the operation button (31A or 31B) is in the ON state. In this pattern, the end effector 18 is provided with a heating element (not illustrated) so that the electrical energy (direct current power or alternating current power) is supplied from the energy source 25 to the heating element. Then, the heat generated by the heating element is transmitted to the end effector 18.

In the pattern Xa2, the medical treatment device 1 functions in a mode in which high frequency energy is transmitted to the end effector 18 in accordance with the fact the selection button 41 is in the OFF state and the operation button (31A or 31B) is in the ON state. The medical treatment device 1 functions in a mode in which heat is transmitted to the end effector 18 in accordance with the fact the selection button 41 is in the ON state and the operation button (31A or 31B) is in the ON state.

In the pattern X3, the medical treatment device 1 functions in a mode in which high frequency energy is transmitted to the end effector 18 in accordance with the fact the selection button 41 is in the OFF state and the operation button (31A or 31B) is in the ON state. The medical treatment device 1 functions in a mode in which ultrasonic vibration is transmitted to the end effector 18 and water is conveyed near the end effector 18 through a water convey path (not illustrated) in accordance with the fact the selection button 41 is in the ON state and the operation button (31A or 31B) is in the ON state. In this pattern, a water convey source (not illustrated) that supplies water (liquid) through the water convey path is provided, and the control unit 26 controls the operation, for example, of a water convey pump of the water convey source. In each mode described in the pattern Xa3, the function to supply high frequency energy to the end effector 18 is added to the functions of the mode corresponding to the pattern X3.

Alternatively, in the pattern X4, the medical treatment device 1 functions in a mode in which ultrasonic vibration is transmitted to the end effector 18 in accordance with the fact the selection button 41 is in the OFF state and the operation button (31A or 31B) is in the ON state. The medical treatment device 1 functions in a mode in which ultrasonic vibration is transmitted to the end effector 18 and suction is performed from the periphery of the end effector 18 through a suction path (not illustrated) in accordance with the fact the selection button 41 is in the ON state and the operation button (31A or 31B) is in the ON state. In this pattern, a suction source (not illustrated) that perform suction through the suction path, and the control unit 26 controls the operation, for example, of a suction pump of the suction source. In each mode in the pattern Xa4, the function to supply high frequency energy to the end effector 18 is added to the functions of the mode corresponding to the pattern X4.

Alternatively, in the pattern X5, the medical treatment device 1 functions in a mode in which ultrasonic vibration is transmitted to the end effector 18 in accordance with the fact the selection button 41 is in the OFF state and the operation button (31A or 31B) is in the ON state. The medical treatment device 1 functions in a mode in which a different type of high frequency energy (the second energy) from the ultrasonic vibration (the first energy) is transmitted to the end effector 18 in accordance with the fact the selection button 41 is in the ON state and the operation button (31A or 31B) is in the ON state. Instead of the supply of high frequency energy in the pattern X5, heat is transmitted to the end effector 18 in the mode in which the medical treatment device 1 functions when the selection button 41 is in the ON state in the pattern Xa5.

The mode in which the medical treatment device 1 functions when the selection button 41 is in the OFF state in the pattern X5 is switched to the mode in which the medical treatment device 1 functions when the selection button 41 is in the ON state in the pattern X6. The mode in which the medical treatment device 1 functions when the selection button 41 is in the ON state in the pattern X5 is switched to the mode in which the medical treatment device 1 functions when the selection button 41 is in the OFF state in the pattern X6. Similarly, the mode in which the medical treatment device 1 functions when the selection button 41 is in the OFF state in the pattern Xa5 is switched to the mode in which the medical treatment device 1 functions when the selection button 41 is in the ON state in the pattern Xa6. The mode in which the medical treatment device 1 functions when the selection button 41 is in the ON state in the pattern Xa5 is switched to the mode in which the medical treatment device 1 functions when the selection button 41 is in the OFF state in the pattern Xa6.

High frequency energy is supplied to the end effector 18 in each mode of the pattern X7 instead of the transmission of ultrasonic vibration in the mode corresponding to the pattern X3. Similarly, high frequency energy is supplied to the end effector 18 in each mode of the pattern X8 instead of the transmission of ultrasonic vibration in the mode corresponding to the pattern X4.

In a mode in which the medical treatment device 1 functions when the selection button 41 is in the OFF state in the pattern X9, the output of energy from the energy source 25 is maintained as long as operation is input with the operation button (31A or 31B). In other words, in a mode in which the medical treatment device 1 functions when the selection button 41 is in the OFF state, the supply of energy to the end effector 18 is maintained as long as the operation button (31A or 31B) is maintained to be in the ON state. When the mode is switched to a mode in which the medical treatment device 1 functions when the selection button 41 is in the ON state, the output of energy is automatically stopped in accordance with the fact that a predetermined period of time T'0 has elapsed since the start of the output of energy from the energy source 25. In other words, in a mode in which the medical treatment device 1 functions when the selection button 41 is in the ON state, the supply of energy to the end effector 18 is stopped when the predetermined period of time T'0 has elapsed since the start of the output of energy even while the operation button (31A or 31B) is maintained to be in the ON state. Even in such a case, however, the output of energy is started in accordance with the fact that the selection button 41 is in the ON state and the operation button (31A or 31B) is in the ON state. Note that, when ultrasonic vibration is transmitted as energy to the end effector 18 and the mode is switched to the mode in which the medical treatment device 1 functions when the selection button 41 is in the ON state, the output of energy can automatically be stopped in accordance with the fact that a predetermined period of time T'1 has elapsed since the start of a phase locked loop control (PLL control).

An incision is detected based on a sound impedance Z in a mode in which the medical treatment device 1 functions when the selection button 41 is in the ON state in the pattern Xa9 instead of stopping the output of energy after the predetermined period of time T'0 has elapsed in the pattern X9. In this pattern, ultrasonic vibration is transmitted as energy to the end effector 18 so that the object to be treated that is grasped between the grasp units 16 and 17 is sectioned with the ultrasonic vibration. The control unit 26 chronologically detects, in the vibration generation unit 21, the sound impedance Z of the electrical energy (alternating current power) supplied to the vibration generation unit 21. When the sound impedance Z exceeds a set threshold Zth, it is determined that the incision of the object to be treated is completed. When it is determined that the incision is completed, the control unit 26 notifies the operator of the completion of incision or the output of energy from the energy source 25 is automatically stopped. This prevents the ultrasonic vibration from wearing the end effector 18 after the completion of incision.

In the pattern X10, the detection of an incision described in the pattern Xa9 is performed both in a mode in which the medical treatment device 1 functions when the selection button 41 is in the OFF state and in a mode in which the medical treatment device 1 functions when the selection button 41 is in the ON state. However, in the pattern X10, the threshold Zth is set at a high value in the mode in which the medical treatment device 1 functions when the selection button 41 is in the OFF state, and the threshold Zth is set at a low value in the mode in which the medical treatment device 1 functions when the selection button 41 is in the ON state in comparison to the mode in which the medical treatment device 1 functions when the selection button 41 is in the OFF state.

In the pattern X11, energy (electrical energy) to be supplied from the energy source 25 to the vibration generation unit 21 at a normal level is output so that ultrasonic vibration is transmitted to the end effector 18 in a mode in which the medical treatment device 1 functions when the selection button 41 is in the OFF state. The level at which the energy is output is lowered in the mode in which the medical treatment device 1 functions when the selection button 41 is in the ON state in comparison with the mode in which the medical treatment device 1 functions when the selection button 41 is in the OFF state so that the amplitude of the ultrasonic vibration is reduced in the end effector 18. In each of the patterns Xa11 to Xc11, high frequency energy is output at a normal level from the energy source 25 in the mode in which the medical treatment device 1 functions when the selection button 41 is in the OFF state so that the high frequency energy is supplied to the end effector 18. In each of the patterns Xa11 to Xc11, the level at which the high frequency energy is output is lowered in the mode in which the medical treatment device 1 functions when the selection button 41 is in the ON state in comparison to the mode in which the medical treatment device 1 functions when the selection button 41 is in the OFF state. This causes the reduction in the high frequency electricity as the pattern Xa11, or the reduction in the maximum value (wave height) of the high frequency voltage as the pattern Xb11, or the reduction in the maximum value (wave height) of the high frequency current as the pattern Xc11.

In contrast to the pattern X11, the level at which the high frequency energy is output is increased in the mode in which the medical treatment device 1 functions when the selection button 41 is in the ON state in comparison to the mode in which the medical treatment device 1 functions when the selection button 41 is in the OFF state so that the amplitude of the ultrasonic vibration in the end effector 18 is increased in the pattern X12. In contrast to the patterns Xa11 to Xc11, the level at which the high frequency energy is output is increased in the mode in which the medical treatment device 1 functions when the selection button 41 is in the ON state in comparison to the mode in which the medical treatment device 1 functions when the selection button 41 is in the OFF state in each of the patterns Xa12 to Xc12. This causes the increase in the high frequency electricity as the pattern Xa12, the increase in the maximum value (wave height) of the high frequency voltage as the pattern Xb12, or the increase in the maximum value (wave height) of the high frequency current as the pattern Xc12.

In the pattern X13, energy (for example, high frequency energy, or the electrical energy to the vibration generation unit 21) is output from the energy source 25 at a level used for treatment in the mode in which the medical treatment device 1 functions when the selection button 41 is in the OFF state (the first mode) so that the energy to be used for treatment (for example, high frequency energy or ultrasonic vibration) is supplied to the end effector 18. In the mode in which the medical treatment device 1 functions when the selection button 41 is in the ON state (the second mode), the measurement unit 28 measures a predetermined parameter. When high frequency energy is supplied to the end effector 18, the predetermined parameter to be measured can be, for example, the phase difference between the high frequency current and the high frequency voltage, the value of the high frequency current, the value of the high frequency voltage, or the chronological variations thereof. When the ultrasonic vibration is transmitted to the end effector 18, the predetermined parameter to be measured can be, for example, the phase difference between the current and voltage of the electrical energy supplied to the vibration generation unit 21, the value of current, the value of voltage, or the chronological variations thereof. When high frequency energy is supplied to the end effector 18, an impedance Z' of the object to be treated that is grasped between the grasp units 16 and 17 can be measured as the predetermined parameter. When the impedance Z' of the object to be treated is measured as the predetermined parameter the object to be treated, the high frequency energy is output from the energy source 25 at a lower level than the level used for the treatment and the high frequency energy is supplied to the end effector 18.

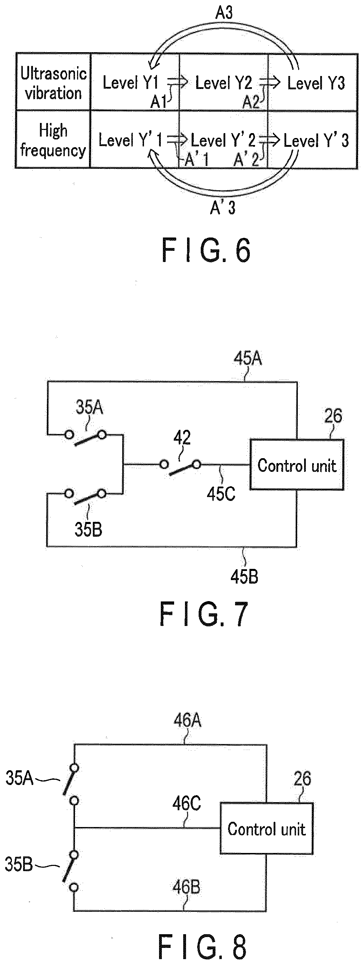

In the first exemplary variation, the mode in which the medical treatment device 1 functions is switched as the illustrated pattern X14 in accordance with the operating state input with the selection button 41. Fig. 6 is an explanatory diagram of a plurality of modes in which the medical treatment device 1 according to the present exemplary variation can function. As illustrated in Figs. 5 and 6, in the pattern X14, energy (for example, high frequency energy, or the electrical energy to the vibration generation unit 21) is output at a set level from the energy source 25 so that energy used for treatment (for example, high frequency energy or ultrasonic vibration) is supplied to the end effector 18 in the mode in which the medical treatment device 1 functions when the selection button 41 is in the OFF state (the first mode). In the mode in which the medical treatment device 1 functions when the selection button 41 is in the ON state (the second mode), the process performed by the level setting unit 27 of the control unit 26 changes the set level of the energy output from the energy source 25.

For example, in an exemplary embodiment illustrated in Fig. 6, ultrasonic vibration is transmitted to the end effector 18 when the mode is switched to a mode in which the medical treatment device 1 functions in accordance with the fact that the selection button 41 is in the OFF state and the operation button 31A is in the ON state. In this example, the energy source 25 supplies the electrical energy at a set level among the levels Y1 to Y3 of to the vibration generation unit 21. In a mode in which the medical treatment device 1 functions in accordance with that fact that the selection button 41 is in the ON state and the operation button 31A is the ON state, the control unit 26 changes the set level of the electrical energy supplied to the vibration generation unit 21 (arrow A1, A2, or A3 in Fig. 6). In this example, only the level of the electrical energy is changed. The electrical energy is not output from the energy source 25 to the vibration generation unit 21.

In the exemplary embodiment illustrated in Fig. 6, high frequency energy is supplied to the end effector 18 when the mode is switched to the mode in which the medical treatment device 1 functions in accordance with the fact the selection button 41 is in the OFF state and the operation button 31B is in the ON state. In this example, the energy source 25 supplies the high frequency energy at a set level among the levels Y'1 to Y'3 to the end effector 18. In a mode in which the medical treatment device 1 functions in accordance with the fact that the selection button 41 is in the ON state and the operation button 31B is the ON state, the control unit 26 changes the set level of the high frequency energy supplied to the end effector 18 (arrow A'1, A'2, or A'3 in Fig. 6). In this example, only the set level of the high frequency energy is changed. The high frequency energy is not output from the energy source 25 to the end effector 18.

When the mode is switched in the pattern X15 in an exemplary variation, energy (for example, high frequency energy or the electrical energy to the vibration generation unit 21) is output at a level used for treatment from the energy source 25 so that the energy used for treatment (for example, high frequency energy or ultrasonic vibration) is supplied to the end effector 18 in the mode in which the medical treatment device 1 functions when the selection button 41 is in the OFF state (the first mode). The energy is supplied to the end effector 18 in a similar manner to the mode in which the medical treatment device 1 functions when the selection button 41 is in the OFF state, and the handle 11 is electrically operated and closed in the mode in which the medical treatment device 1 functions when the selection button 41 is in the ON state (the second mode). In this example, the inside of the housing 6 is provided with an electric motor (not illustrated) so that electrical energy (drive electricity) is supplied from the energy source 25 to the electric motor. This electricity drives the electric motor. Driving the electric motor makes driving force act on the handle 11 so that the handle 11 is closed.

The process in accordance with the operating state input with the operation button (31A or 31B) is not limited to the process illustrated in Fig. 3 and the process performed in a similar manner to Fig. 3. For example, when the selection button 41 is in the OFF state and the operation button (31A or 31B) is switched to the ON state (step S101-Yes and S102-Yes in Fig. 3) and the selection button 41 is switched from the OFF state to the ON state before the given period of time T0 has elapsed since the operation button (31A or 31B) has been switched to the ON state (step S103-No in Fig. 3), an error is warned and a state in which the medical treatment device 1 is stopped from functioning is maintained in the first embodiment. However, when the selection button 41 is switched from the OFF state to the ON state before the given period of time T0 has elapsed since the operation button (31A or 31B) has been switched to the ON state, it is determined in an exemplary variation that the operation button (31 A or 31B) and the selection button 41 are simultaneously in the ON state. Then, the control unit 26 causes the medical treatment device 1 to function in a mode in which the medical treatment device 1 functions in accordance with the fact that the operation button (31 A or 31B) and the selection button 41 are simultaneously in the ON state (for example, the second mode).

In an exemplary variation, as long as the control by the control unit 26 maintains the operation button (31A or 31B) in the ON state, the mode in which the medical treatment device 1 functions is not changed even when the operating state is switched with the selection button 41 (the selection button 41 is switched between the ON state and the OFF state). To switch the mode in which the medical treatment device 1 functions in this example, it is necessary to switch the operation button (31A or 31B) from the OFF state to the ON state after the operating state input with the selection button 41 is switched while the operation button (31A or 31B) is in the OFF state. In contrast, in another exemplary variation, the control by the control unit 26 switches the operating state input with the selection button 41 (switches the selection button 41 between the ON state and the OFF state). This switching changes the mode in which the medical treatment device 1 functions even while the operation button (31A or 31B) is in the ON state.

In the second exemplary variation, the mode in which the medical treatment device 1 functions is switched to the mode as the pattern X16 illustrated in Fig. 5 in accordance with the operating state input with the selection button 41. In the pattern X16, the medical treatment device 1 operates in accordance with the operation input with the foot switch (the operation input apparatus) 34 in the mode in which the medical treatment device 1 functions when the selection button 41 is in the OFF state (the first mode). In other words, when the selection button 41 is in the OFF state, the medical treatment device 1 operates in accordance with the operation input with the foot switch 34 regardless of the operation input with the operation button (31A or 31B) (whether the operation button (31A or 31B) is in the ON state or the OFF state). For example, when operation is input with the foot switch 34, energy (for example, high frequency energy, or the electrical energy to the vibration generation unit 21) is output from the energy source 25 so that the energy used for treatment (for example, high frequency energy or ultrasonic vibration) is supplied to the end effector 18.

On the other hand, in the pattern X16, the medical treatment device 1 operates in accordance with the operation input with the operation button (the operation member) 31A or 31B in the mode in which the medical treatment device 1 functions when the selection button 41 is in the ON state (the second mode). In other words, when the selection button 41 is in the ON state, the medical treatment device 1 operates in accordance with the operation input with the operation button (31A or 31B) regardless of the operation input with the foot switch 34. For example, when operation is input with the operation button 31A, energy (for example, high frequency energy, or the electrical energy to the vibration generation unit 21) is output from the energy source 25 so that the energy used for treatment (for example, high frequency energy or ultrasonic vibration) is supplied to the end effector 18.

Fig. 7 is a diagram of the configuration to detect the operating state input with each of the operation buttons 31A and 31B, and the selection button 41 in the present exemplary variation. In the present exemplary variation, the operating state input with the operation button corresponding to each of the switches 35A and 35B (the operation button 31A or 31B) is detected in accordance with whether each of the switches 35A and 35B opens or closes, and the operating state input with the selection button 41 is detected in accordance with whether the switch 42 opens or closes. As illustrated in Fig. 7, in the present exemplary variation, the control unit 26 is connected to the switch 35A through the detection signal line 45A, and the control unit 26 is connected to the switch 35B through the detection signal line 45B. Similarly, the control unit 26 is connected to the switch 42 through the detection signal line 45C. In the present exemplary variation, the switches 35A and 42 are electrically arranged in series each other and the switches 35B and 42 are electrically arranged each other. The detection signal lines 45A to 45C are a first detection signal line that transmits a detection signal (first detection signal) indicating the operating state input with each of the operation buttons 31A and 31B to the control unit 26, and are a second detection signal line that transmits a detection signal (second detection signal) indicating the operating state input with the selection button 41 to the control unit 26.

In the present exemplary variation, when both the operation button 31A and the selection button 41 are in the ON states, both the switches 35A and 42 are closed and thus the detection current flows through the detection signal lines 45A and 45C. The flow of the detection current through the detection signal lines 45A and 45C switches the mode to a mode in which the medical treatment device 1 operates in accordance with the operation input with the operation button 31A and, for example, energy used for treatment is transmitted to the end effector 18 in accordance with the operation input with the operation button 31A. When the selection button 41 is in the OFF state, the detection current does not flow through the detection signal lines 45A and 45C even when the operation button 31A is in the ON state. Thus, when the selection button 41 is in the OFF state, the medical treatment device 1 operates in accordance with the operation input with the foot switch 34 even when the operation button 31A in the ON state. Accordingly, when the operation button 31A and the selection button 41 are momentary ones, the medical treatment device 1 operates in accordance with the operation input with the foot switch 34 unless operation is simultaneously input with the operation button 31A and the selection button 41 (unless the operation button 31A and the selection button 41 are simultaneously pressed) even when the operation is input with the operation button 31A (the operation button 31A is pressed).

In the present exemplary variation, when both the operation button 31B and the selection button 41 are in the ON states, both the switches 35B and 42 are closed and thus the detection current flows through the detection signal lines 45B and 45C. The flow of the detection current through the detection signal lines 45B and 45C switches the mode to a mode in which the medical treatment device 1 operates in accordance with the operation input with the operation button 31B and, for example, energy used for treatment is transmitted to the end effector 18 in accordance with the operation input with the operation button 31B. When the selection button 41 is in the OFF state, the detection current does not flow through the detection signal lines 45B and 45C even when the operation button 31B is in the ON state. Thus, when the selection button 41 is in the OFF state, the medical treatment device 1 operates in accordance with the operation input with the foot switch 34 even when the operation button 31B is the ON state. Accordingly, when the operation button 31B and the selection button 41 are momentary ones, the medical treatment device 1 operates in in accordance with the operation input with the foot switch 34 unless the operation is simultaneously input with the operation button 31B and the selection button 41 (unless the operation button 31B and the selection button 41 are simultaneously pressed) even when operation is input with the operation button 31B (even when the operation button 31B is pressed).

Fig. 8 is a diagram of the configuration to detect the operating state input with each of the operation buttons 31A and 31B in a third exemplary variation. In the present exemplary variation, the operating state input with the operation button corresponding to each of the switches 35A and 35B (the operation button 31A or 31B) is also detected in accordance with whether each of the switches 35A and 35B opens or closes. As illustrated in Fig. 8, in the present exemplary variation, the control unit 26 is connected to the switch 35A through the detection signal line 46A, and the control unit 26 is connected to the switch 35B through the detection signal line 46B. Similarly, the control unit 26 is connected to the switches 35A and 35B through the detection signal line 46C. The detection signal lines (first detection signal lines) 46A to 46C transmits a detection signal (first detection signal) indicating the operating state input with each of the operation buttons 31A and 31B to the control unit 26.

In the present exemplary variation, when the operation button 31A is in the ON state and the operation button 31B is in the OFF state, the detection current flows through the detection signal lines 46A and 46B. When the operation button 31A is in the OFF state and the operation button 31B is in the ON state, the detection current flows through the detection signal lines 46B and 46C. When both the operation buttons 31A and 31B are in the ON states, the detection current flows through the detection signal lines 46A and 46B. Thus, both when the selection button 41 is in the OFF state and when the selection button 41 is in the ON state in the present exemplary variation, the medical treatment device 1 can function in three modes; a mode in which only the operation button 31A is in the ON state, a mode in which only the operation button 31B is in the ON state, and a mode in which both the operation buttons 31A and 31B are in the ON states. In other words, both when the selection button 41 is in the OFF state and when the selection button 41 is in the ON state, the medical treatment device 1 can function in a larger number of modes than the number of the operation buttons 31A and 31B that are the operation members.

In an exemplary embodiment, when the selection button 41 is in the ON state and only the operation button 31A is in the ON state, ultrasonic vibration is supplied to the end effector 18. Alternatively, when the selection button 41 is in the ON state and only the operation button 31B is in the ON state, high frequency energy is supplied to both the grasp units 16 and 17 of the end effector 18 so that a bipolar treatment by the high frequency energy is performed. Alternatively, when the selection button 41 is in the ON state and both the operation buttons 31A and 31B are in the ON states, high frequency energy is supplied to only one of the grasp units 16 and 17 of the end effector 18 so that a mono-polar treatment by the high frequency energy is performed.

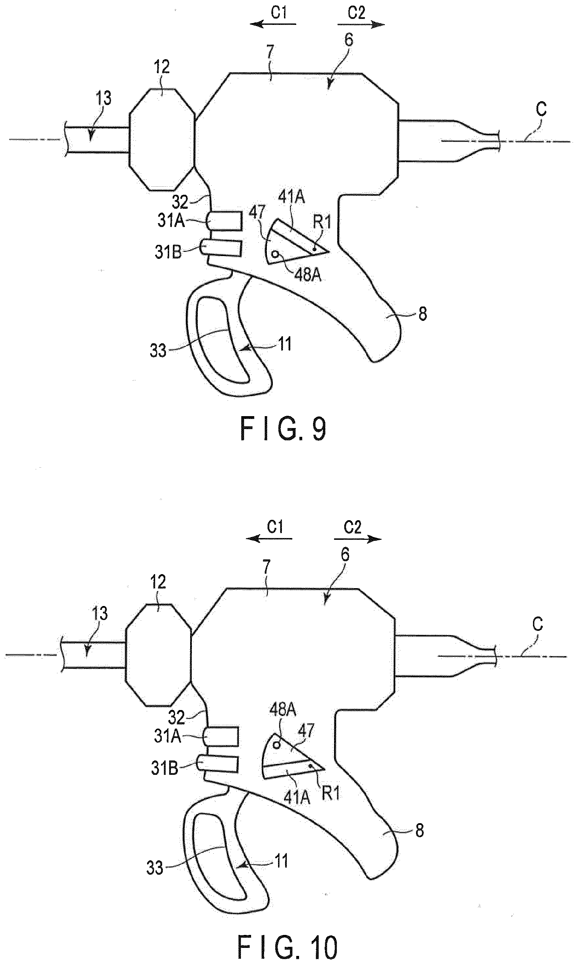

In the embodiments, when the selection button 41 that is a selection member is an alternate button, the selection button 41 is maintained to be in both the ON state and the OFF state. However, the selection member is not limited to the alternate button. For example, the external surface of the housing 6 is provided with a selection lever 41A as the selection member in a fourth exemplary variation illustrated in Figs. 9 to 11. The selection lever 41A is placed at a portion facing a first end of the housing 6 in the width direction of the housing 6 (the direction perpendicular to the drawing papers of Figs. 9 and Fig. 10) on the external surface of the housing 6. The external surface of the housing 6 is provided with a concavity 47 in the present exemplary variation so that the selection lever 41A can rotate around a rotation shaft R1 in the concavity 47. The selection lever 41A can rotate between an OFF state position (the position illustrated in Fig. 9) and an ON state position (the position illustrated in Fig. 10).

In the present exemplary variation, the selection lever 41A is also placed nearer to the base side of the housing 6 than the operation buttons 31A and 31B. Thus, the thumb F1 can input operation with the selection lever 41A (can rotate the selection button 41A) while the grip 8 is grasped between the thumb F1 and the palm P. The thumb F1 switches the selection lever 41A between the ON state and the OFF state.

In the present exemplary variation, the external surface of the housing 6 is provided with engagement protrusions 48A and 48B in the concavity 47. An engagement groove 49, which can be engaged with the engagement protrusions 48A and 48B, is formed on the selection lever 41A. The engagement of the engagement groove 49 with the engagement protrusion 48A fixes the selection lever 41A on the OFF state position with respect to the housing 6 so that the selection lever 41A that is the selection member is maintained to be in the OFF state. In other words, the engagement protrusion 48A and the engagement groove 49 work as a state maintenance mechanism that maintains the selection lever 41A in the OFF state. The engagement of the engagement groove 49 with the engagement protrusion 48B fixes the selection lever 41A on the ON state position with respect to the housing 6 so that the selection lever 41A that is the selection member is maintained to be in the ON state. In other words, the engagement protrusion 48B and the engagement groove 49 work as a state maintenance mechanism that maintains the selection lever 41A in the ON state.

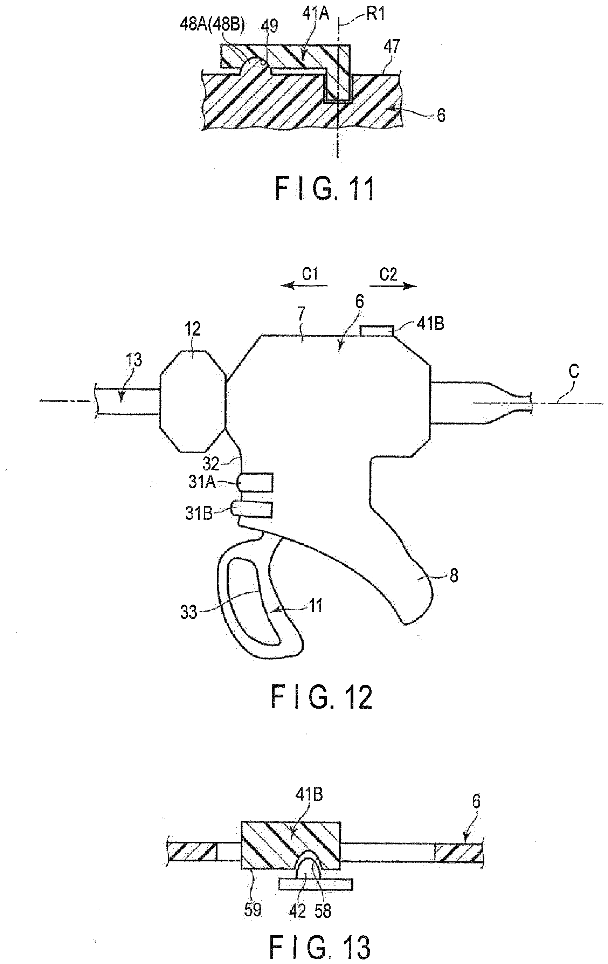

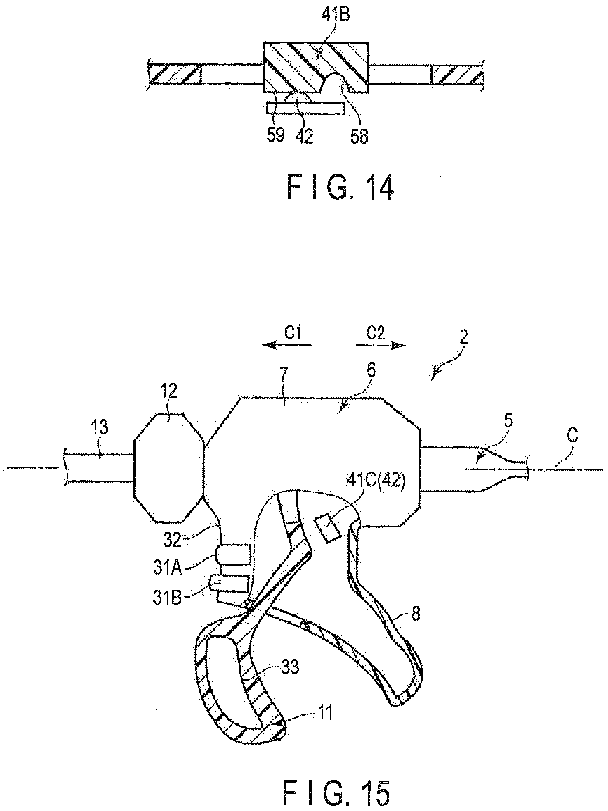

In a fifth exemplary variation illustrated in Figs. 12 to 14, the external surface of the housing 6 is provided with a selection slider 41B as the selection member. The selection slider 41B is placed at a portion facing a side opposite to the side on which the force application unit 33 is placed with respect to the longitudinal axis C (the upper side of Fig. 12) on the external surface of the housing 6. In the present exemplary variation, the selection slider 41B can move in the direction along the longitudinal axis C with respect to the housing 6. Moving the selection slider 41B switches the selection slider 41B between the OFF state (the state illustrated in Fig. 13) and the ON state (the state illustrated in Fig. 14).

In the present exemplary variation, the selection slider 41B is placed nearer to the base side of the housing 6 than the operation buttons 31A and 31B. Thus, the thumb F1 can input operation with the selection slider 41B (can move the selection slider 41B) while the grip 8 is grasped between the thumb F1 and the palm P. The thumb F1 switches the selection slider 41B between the ON state and the OFF state.

In the present exemplary variation, the internal surface of the selection slider 41B is provided with a groove 58 and a pressure unit 59. As illustrated in Fig. 13, moving the selection slider 41B to a position at which the switch 42 is inserted into the groove 58 prevents the selection slider 41B from pressing the switch 42. Thus, the switch 42 is maintained to be in an opening state and the selection slider 41B is maintained to be in the OFF state. In other words, the groove 58 works as a state maintenance mechanism that maintains the selection slider 41B in the OFF state. When the selection slider 41B is moved to a position at which the pressure unit 59 presses the switch 42, the pressure by the pressure unit 59 maintains the switch 42 in a closing state. This maintains the selection slider 41B in the ON state. In other words, the pressure unit 59 works as a state maintenance mechanism that maintains the selection slider 41B in the ON state.

In the embodiments described above, the selection member (41, 41A, or 41B) is placed on the external surface of the housing 6. However, the placement is not limited to the embodiments. For example, in a sixth exemplary variation illustrated in Fig. 15, the inside of the housing 6 is provided with a selection switch 41C including a switch 42 as the selection member. In the present exemplary variation, the selection switch 41C is placed nearer to the base side of the housing 6 than the operation buttons 31A and 31B. In the present exemplary variation, the selection switch 41C is placed on a position at which the handle 11 can press the selection switch 41C so that the handle 11 opens or closes with respect to the housing 6. This opening or closing varies the pressure from the handle 11 on the selection switch 41C. The variation in the pressure from the handle 11 on the selection switch 41C switches the selection switch 41C between the ON state and the OFF state (in other words, the variation switches the operating states input with the selection switch 41C). Note that switching the operating states input with the selection switch 41C switches the switch 42 between the opening and closing states.

For example, when the selection switch 41C is a momentary one, closing the handle 11 with respect to the grip 8 causes the handle 11 to press the selection switch 41C so that the selection switch 41C is switched to the ON state. Opening the handle 11 with respect to the grip 8 prevents the handle 11 from having contact with the selection switch 41C so that the selection switch 41C is switched to in the OFF state. In the present exemplary variation, the control unit 26 also causes the medical treatment device 1 to function, for example, in one of the patterns X1 to X16, Xa1 to Xa6, Xa9, Xa11 to Xc11, and Xa12 to Xc12 illustrated in Fig. 5 in accordance with the operating state input with the selection switch 41C.

In the present exemplary variation, the selection switch (the selection member) 41C is placed nearer to the base side of the housing 6 than the operation buttons 31A and 31B in the housing 6. Thus, opening or closing the handle 11 with respect to the housing 6 can vary the pressure from the handle 11 on the selection switch 41C. In other words, in the present exemplary variation, opening or closing the handle 11 simultaneously switches the selection switch 41C between the ON state and the OFF state. In the present exemplary variation, the housing 6 and the handle 11 are held as described in the first embodiment (see Fig. 4). Thus, it is unnecessary in the present exemplary variation to change the positions and postures of the operator's index finger F2, middle finger F3, ring finger F4, and little finger F5 and to hold the housing 6 and the handle 11 again with the operator's right hand H during the input of operation with the selection switch 41C because opening or closing the handle 11 switches the operating states input with the selection switch 41C. Thus, in the present exemplary variation, the operability for opening and closing the handle 11, rotating the rotating knob 12, inputting operation with the operation buttons 31A and 31B is secured and the operability for inputting operation with the selection switch 41C is also secured.

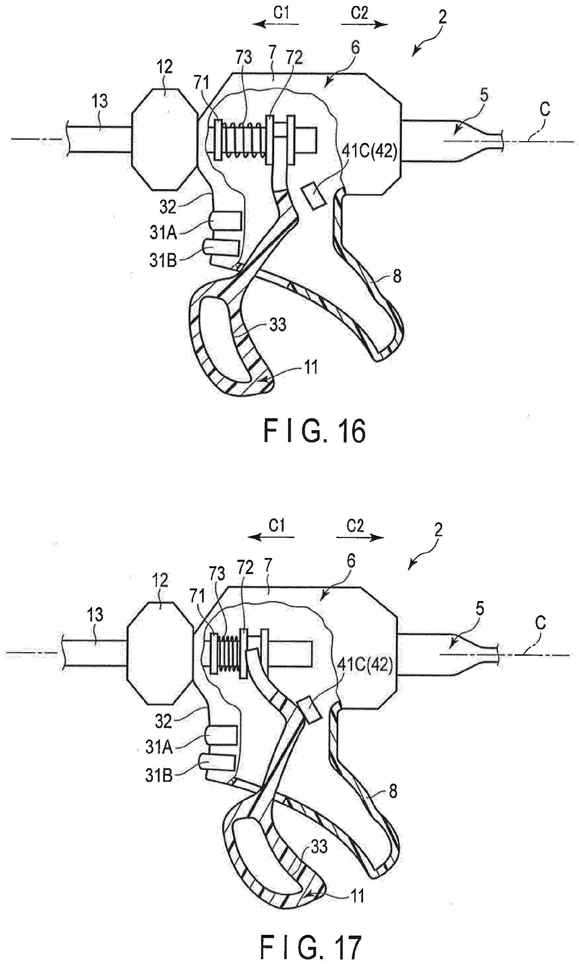

In a seventh exemplary variation illustrated in Figs. 16 and 17, opening and closing the handle 11 with respect to the housing 6 varies the pressure from the handle 11 on the selection switch 41C. The variation in pressure from the handle 11 on the selection switch 41C switches the selection switch 41C between the OFF state (the state illustrated in Fig. 16) and the ON state (the state illustrated in Fig. 17).

In the present exemplary variation, a movable tube 71 extends along the longitudinal axis C in the housing body 7. The movable tube 71 moves together with a movable pipe (not illustrated) along the longitudinal axis C in response to the opening or closing of the handle 11. A ring-shaped sliding member 72 is placed on the outer peripheral surface of the movable tube 71. The handle 11 is coupled with the sliding member 72 in the housing body 7. The sliding member 72 can move along the longitudinal axis C with respect to the movable tube 71. A tube-shaped elastic member (coil spring) 73 is also placed on the outer peripheral surface of the movable tube 71. A first end (the head) of the elastic member 73 is connected to the movable tube 71 and a second end (the base) of the elastic member 73 is connected to the sliding member 72.

In the present exemplary variation, closing the handle 11 with respect to the grip 8 when the selection switch 41C that is the selection member is in the OFF state moves the movable tube 71 and the movable pipe toward the head side so that the grasp units 16 and 17 are closed. Thus, the object to be treated is grasped between the grasp units 16 and 17 so that the object to be treated is compressed. When the object to be treated is compressed to some extent, the motion of the movable tube 71 and the movable pipe stops. Further closing the handle 11 from that state moves the sliding member 72 toward the head side with respect to the movable tube 71. This causes the elastic member 73 to contract. This contraction of the elastic member 73 increases the holding force between the grasp units 16 and 17.

In the present exemplary variation, the elastic member 73 contracts by a predetermined contraction amount (a predetermined stroke) or more. This contraction causes the handle 11 to press the selection switch 41C (the switch 42) and thus the selection switch 41C is switched from the OFF state to the ON state. Thus, even when the handle 11 is closed, the selection switch 41C is maintained to be in the OFF state unless the application of operation force in an amount more than or equal to a predetermined amount causes the elastic member 73 to contract by a predetermined contraction amount or more. Thus, in the present exemplary variation, the selection switch 41C is switched to the ON state only when the object to be treated is grasped between the grasp units 16 and 17 and the force application unit 33 applies the operation force more than or equal to a predetermined amount of force and the handle 11 is closed.

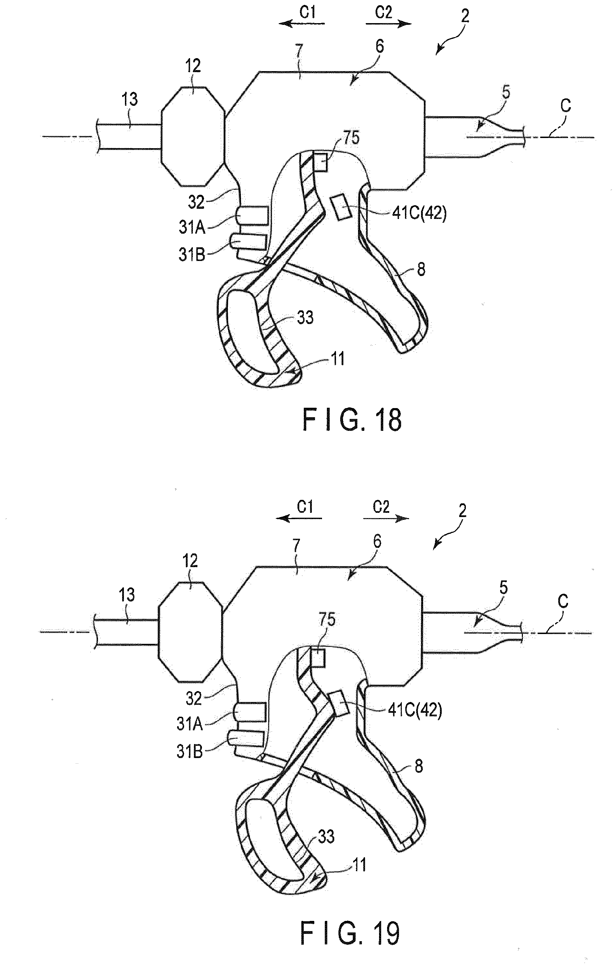

In an eighth exemplary variation illustrated in Figs. 18 and 19, opening or closing the handle 11 with respect to the housing 6 varies the pressure from the handle 11 on the selection switch 41C. The variation in pressure from the handle 11 on the selection switch 41C switches the selection switch 41C between the OFF state (the state illustrated in Fig. 18) and the ON state (the state illustrated in Fig. 19). In the present exemplary variation, the inside of the housing 6 is provided with a stopper 75. The stopper 75 is fixed with respect to the housing 6.

In the present exemplary variation, closing the handle 11 with respect to the grip 8 when the selection switch 41C that is the selection member is in the OFF state causes the handle 11 to have contact with the stopper 75. Further closing the handle 11 from that state causes the handle 11 to warp on the contact position with the stopper 75 as a fulcrum.

In the present exemplary variation, the handle 11 warps by a predetermined warping amount or more after the handle 11 comes into contact with the stopper 75. This warping causes the handle 11 to press the selection switch 41C (the switch 42), and thus switches the selection switch 41C from the OFF state to the ON state. Thus, even when the handle 11 is closed, the selection switch 41C is maintained to be in the OFF state unless the application of operation force in an amount more than or equal to a predetermined amount causes the handle 11 to warp by a predetermined warping amount or more. Thus, similarly to the seventh exemplary variation, the selection switch 41C is switched to the ON state only when the object to be treated is grasped between the grasp units 16 and 17 and the force application unit 33 applies the operation force in an amount more than or equal to a predetermined amount and the handle 11 is closed in the present exemplary variation.

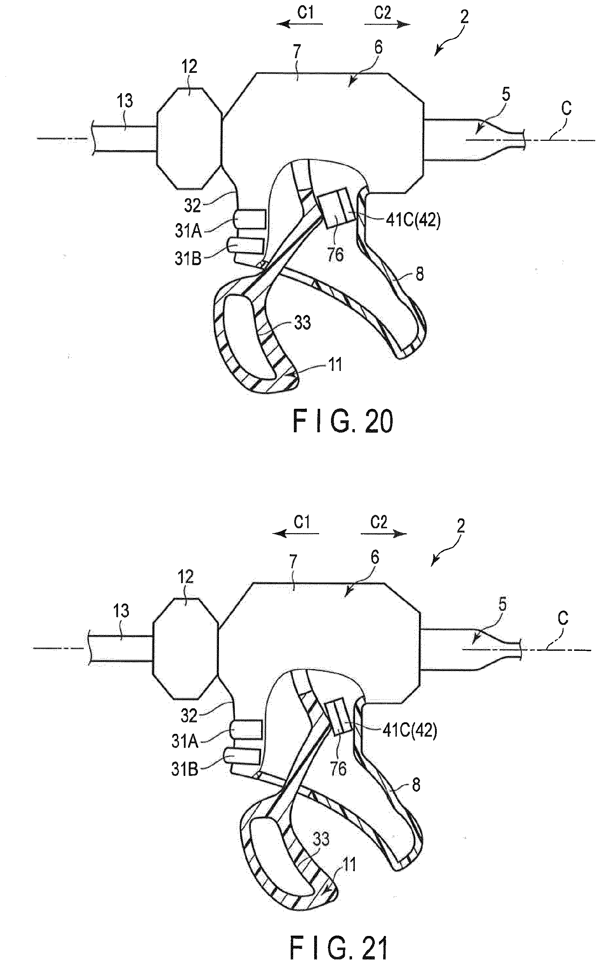

In a ninth exemplary variation illustrated in Figs. 20 and 21, opening or closing the handle 11 with respect to the housing 6 varies the pressure from the handle 11 on the selection switch 41C. The variation in pressure from the handle 11 on the selection switch 41C switches the selection switch 41C between the OFF state (the state illustrated in Fig. 20) and the ON state (the state illustrated in Fig. 21). In the present exemplary variation, the inside of the housing 6 is provided with an elastic member 76. The selection switch 41C is attached on the elastic member 76.

In the present exemplary variation, closing the handle 11 with respect to the grip 8 when the selection switch 41C that is the selection member is in the OFF state causes the handle 11 to have contact with the elastic member 76. Further closing the handle 11 from that state causes the elastic member 76 to contract.

In the present exemplary variation, when the elastic member 76 contracts by a predetermined contraction amount or more, this contraction causes the handle 11 to press the selection switch 41C (the switch 42) and thus switches the selection switch 41C from the OFF state to the ON state. Thus, even when the handle 11 is closed, the selection switch 41C is maintained to be in the OFF state unless the application of operation force in an amount more than or equal to a predetermined amount causes the elastic member 76 to contract by a predetermined contraction amount or more. Thus, similarly to the seventh exemplary variation and the eighth exemplary variation, the selection switch 41C is switched to the ON state only when the object to be treated is grasped between the grasp units 16 and 17 and the force application unit 33 applies the operation force in an amount more than or equal to a predetermined amount and the handle 11 is closed in the present exemplary variation.

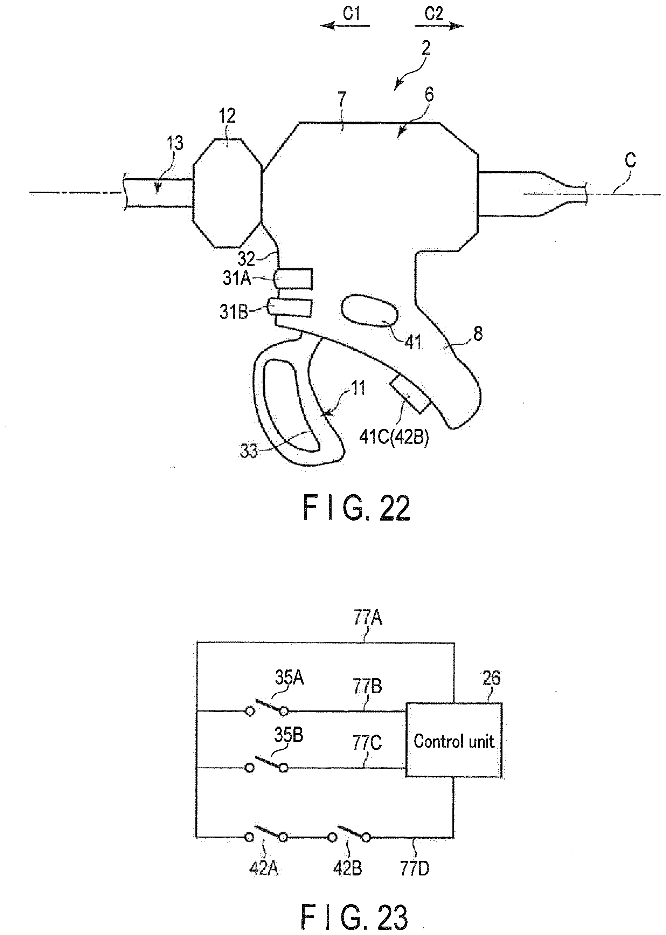

The tenth exemplary variation illustrated in Figs. 22 and 23 is provided with a selection button 41 and a selection switch 41C as selection members. In the present exemplary variation, the selection button 41 is provided nearer to the base side of the housing 6 than the operation buttons 31A and 31B. Thus, the thumb F1 can input operation with the selection button 41 (can press the selection button 41) while the grip 8 is grasped between the thumb F1 and the palm P. The thumb F1 switches the selection button 41 between the ON state and the OFF state. In the present exemplary variation, the selection switch 41C is provided nearer to the base side of the housing 6 than the operation buttons 31A and 31B. Opening or closing the handle 11 with respect to the housing 6 varies the pressure from the handle 11 on the selection switch 41C. The variation in pressure from the handle 11 to the selection switch 41C switches the selection switch 41C between the OFF state and the ON state.

Fig. 23 is a diagram of the configuration to detect the operating state of each of the operation buttons 31A and 31B, the selection button 41, and the selection switch 41C in the present exemplary variation. In the present exemplary variation, the operating state input with the operation button corresponding to each of the switches 35A and 35B (the operation button 31A or 31B) is detected in accordance with whether each of the switches 35A and 35B opens or closes. Furthermore, the operating state input with the selection button 41 is detected in accordance with whether the switch 42A opens or closes and the operating state input with the selection switch 41C is detected in accordance with whether the switch 42B placed on the selection switch 41C opens or closes in the present exemplary variation. As illustrated in Fig. 23, in the present exemplary variation, the control unit 26 is connected to the switches 35A, 35B, and 42A through the detection signal line 77A. The control unit 26 is connected to the switch 35A through the detection signal line 77B, and connected to the switch 35B through the detection signal line 77B. The control unit 26 is also connected to the switch 42B through the detection signal line 77C. In the present exemplary variation, the switches 42A and 42B are electrically arranged in series each other. The detection signal lines 77A to 77D are a first detection signal line that transmits a detection signal (a first detection signal) indicating the operating state input with each of the operation buttons 31A and 31B to the control unit 26, and are a second detection signal line that transmits a detection signal (a second detection signal) indicating the operating state input with each of the selection button 41 and the selection switch 41C to the control unit 26.

In the present exemplary variation, when both the selection button 41 and the selection switch 41C are in the ON states, both the switches 42A and 42B are closed so that the detection current flows through the detection signal lines 77A and 77D. The flow of the detection current through the detection signal lines 77A and 77D switches the mode from the mode in which both the selection buttons 41 and the selection switch 41C in the OFF states. Thus, even when both the selection button 41 and the selection switch 41C are in the OFF states and one of the selection button 41 and the selection switch 41C is switched to the ON state (even when one of the switch 42A and 42B is closed), the detection current does not flow through the detection signal lines 77A and 77D and thus the mode is not switched.

For example, in an exemplary embodiment, the mode is switched so that the medical treatment device 1 functions as the pattern X16 illustrated in Fig. 5 in accordance with the operating state input with each of the selection button 41 and the selection switch 41C. In this example, when at least one of the selection button 41 and the selection switch 41C is in the OFF state, the detection current does not flow through the detection signal lines 77A and 77D. Thus, the medical treatment device 1 operates in accordance with the operation input with the foot switch 34. For example, even when closing the handle 11 switches the selection switch 41C to the ON state, the medical treatment device 1 operates in accordance with the operation input with the foot switch 34 unless operation is simultaneously input with both the selection button 41 and the selection switch 41C (unless both the selection button 41 and the selection switch 41C are simultaneously pressed). Thus, even when the selection switch 41C is switched to the ON state, the energy used for treatment (for example, high frequency energy or ultrasonic vibration) is not supplied to the end effector 18 in accordance with the operation input with the operation button (31A or 31B) unless both the selection button 41 and the selection switch 41C are in the ON states. In the present exemplary variation, for example, when closing the handle 11 switches the selection switch 41C to the ON state, and operation is simultaneously input with both the selection button 41 and the selection switch 41C (both the selection button 41 and the selection switch 41C are simultaneously pressed), the mode is switched to the mode in which the medical treatment device 1 operates in accordance with the operation input with the operation buttons 31A and 31B.

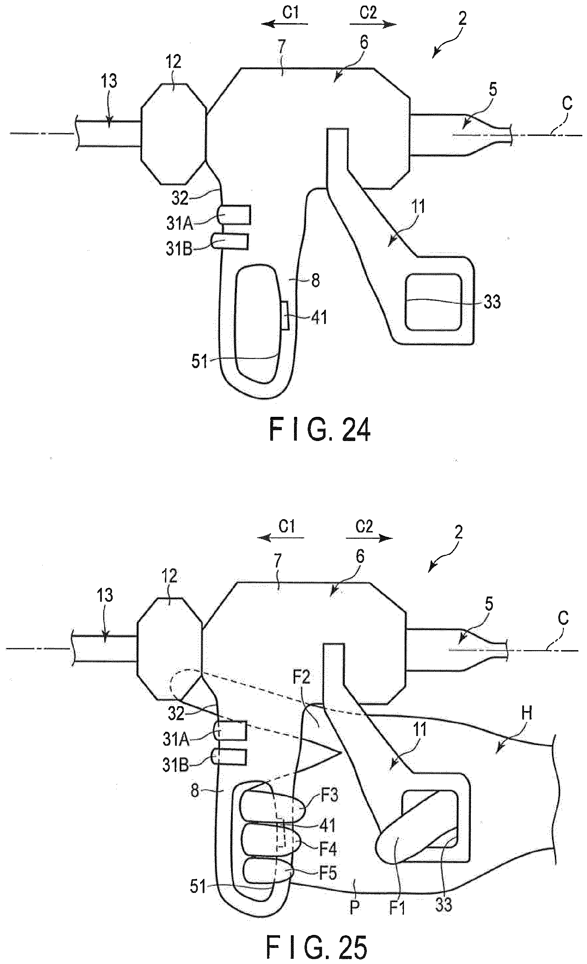

In the embodiments and variations, the handle 11 is placed on the head side of the grip 8. However, as an eleventh exemplary variation illustrated in Figs. 24 and 25, the handle 11 can be placed on the base side of the grip 8. In the present exemplary variation, the handle 11 also opens and closes in roughly parallel to the longitudinal axis C. The grip 8 includes a grip grasp unit (grip finger-put position) 51. In the present exemplary variation, the operation buttons (the operation members) 31A and 31B are placed on the surface of the head 32 of the grip 8 and the operation buttons 31A and 31B are placed nearer to the longitudinal axis C than the grip grasp unit 51. Thus, the operation buttons 31A and 31B are placed at a portion on the head side of the housing 6.

In the present exemplary variation, the grip grasp unit 51 of the grip 8 is provided with a selection button (the selection member) 41. In the present exemplary variation, the selection button 41 is exposed to the outside of the external surface of the housing 6, and placed nearer to the base side of the housing 6 than the operation buttons 31A and 31B. In the present exemplary variation, the control unit 26 causes the medical treatment device 1 to function, for example, in one of the patterns X1 to X16, Xa1 to Xa6, Xa9, Xa11 to Xc11, and Xa12 to Xc12 illustrated in Fig. 5 in accordance with the operating state input with the selection button 41.

As illustrated in Fig. 25, for example, in a state in which an operator holds the housing 6 and the handle 11 with the operator's right hand H, the operator's middle finger F3, ring finger F4 and little finger F5 are put on the grip grasp unit 51 so that the grip 8 of the housing 6 is held. Then, the thumb F1 is put on the force application unit 33 of the handle 11 so that the thumb F1 applies operation force on the handle 11 to close the handle 11 with respect to the grip 8. The index finger F2 performs the operation for rotating the rotating knob 12 and inputting operation with the operation buttons 31A and 31B (pressing the operation buttons 31A and 31B).

In the present exemplary variation, the selection button (the selection member) 41 is placed nearer to the base side of the housing 6 than the operation buttons 31A and 31B on the external surface of the housing 6, and is positioned on the grip grasp unit 51. This enables the operator to input operation with the selection button 41 (to press the selection button 41) using any one of the middle finger F3, ring finger F4, and little finger F5 put on the grip grasp unit 51 of the grip 8. The middle finger F3, ring finger F4, or little finger F5 switches the selection button 41 between the ON state and the OFF state. Thus, the thumb F1 and index finger F2 used for at least one of the operations for opening or closing the handle 11, for rotating the rotating knob 12, and for inputting operation with the operation buttons 31A and 31B are not used for inputting operation with the selection button 41. Thus, it is unnecessary to change the positions and postures of the thumb F1 and index finger F2 and to hold the housing 6 and the handle 11 again with the right hand H in order to switch the selection button 41 between the ON state and the OFF state. Thus, in the present exemplary variation, the operability for opening or closing the handle 11, for rotating the rotating knob 12, and for inputting operation with the operation buttons 31A and 31B is secured and the operability for inputting operation with the selection button 41 is also secured.

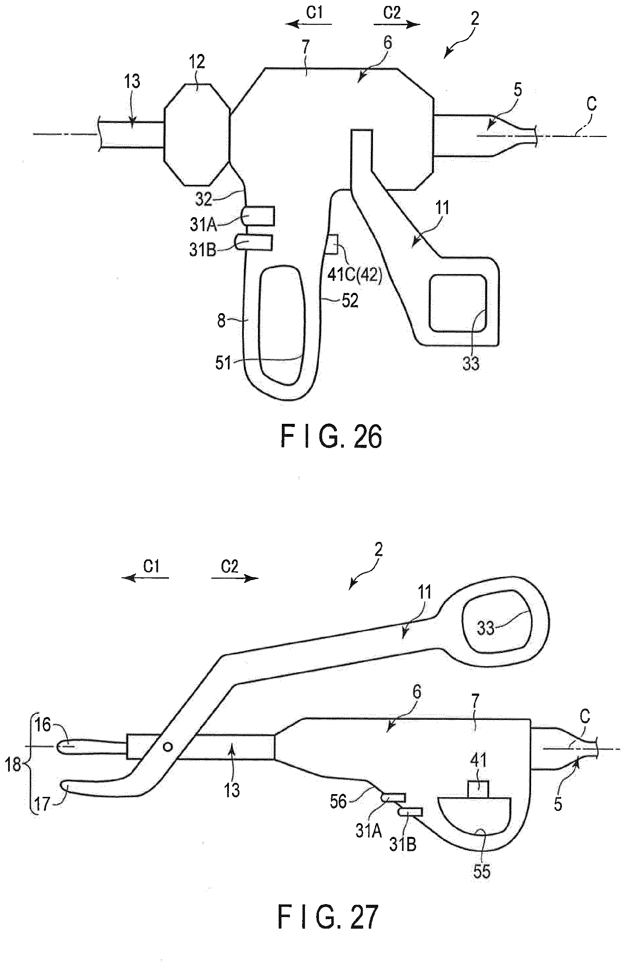

In a twelfth exemplary variation illustrated in Fig. 26, the base side of the grip 8 is provided with the handle 11 and the base surface 52 of the grip 8 is provided with the selection switch 41C (the switch 42). In the present exemplary variation, the selection switch 41C is placed nearer to the base side of the housing 6 than the operation buttons 31A and 31B. Then, the selection switch 41C is exposed to the outside of the external surface of the housing 6. In the present exemplary variation, the selection switch 41C is placed at a position at which the handle 11 can press the selection switch 41C so that opening or closing the handle 11 with respect to the housing 6 varies the pressure from the handle 11 on the selection switch 41C. The variation in pressure from the handle 11 on the selection switch 41C switches the selection switch 41C between the ON state and the OFF state (in other words, the operating state input with the selection switch 41C is switched). In the present exemplary variation, the control unit 26 causes the medical treatment device 1 to function, for example, in one of the patterns X1 to X16, Xa1 to Xa6, Xa9, Xa11 to Xc11, and Xa12 to Xc12 illustrated in Fig. 5 in accordance with the operating state input with the selection switch 41C.

In the present exemplary variation, the selection switch (the selection member) 41C is placed nearer to the base side of the housing 6 than the operation buttons 31A and 31B on the housing 6, and placed on the base surface 52 of the grip 8. Thus, opening or closing the handle 11 with respect to the housing 6 can vary the pressure from the handle 11 on the selection switch 41C. In other words, in the present exemplary variation, opening or closing the handle 11 immediately switches the selection switch 41C between the ON state and the OFF state. In the present exemplary variation, the housing 6 and the handle 11 are held as described in the eleventh exemplary variation (see Fig. 25). Thus, it is unnecessary to change the positions and postures of the thumb F1 and index finger F2 and to hold the housing 6 and the handle 11 again with the right hand H in order to input operation with the selection switch 41C because opening or closing the handle 11 immediately switches the selection switch 41C between the ON state and the OFF state. Thus, in the present exemplary variation, the operability for opening and closing the handle 11, for rotating the rotating knob 12, and for inputting operation with the operation buttons 31A and 31B is secured and the operability for inputting operation with the selection switch 41C is also secured.

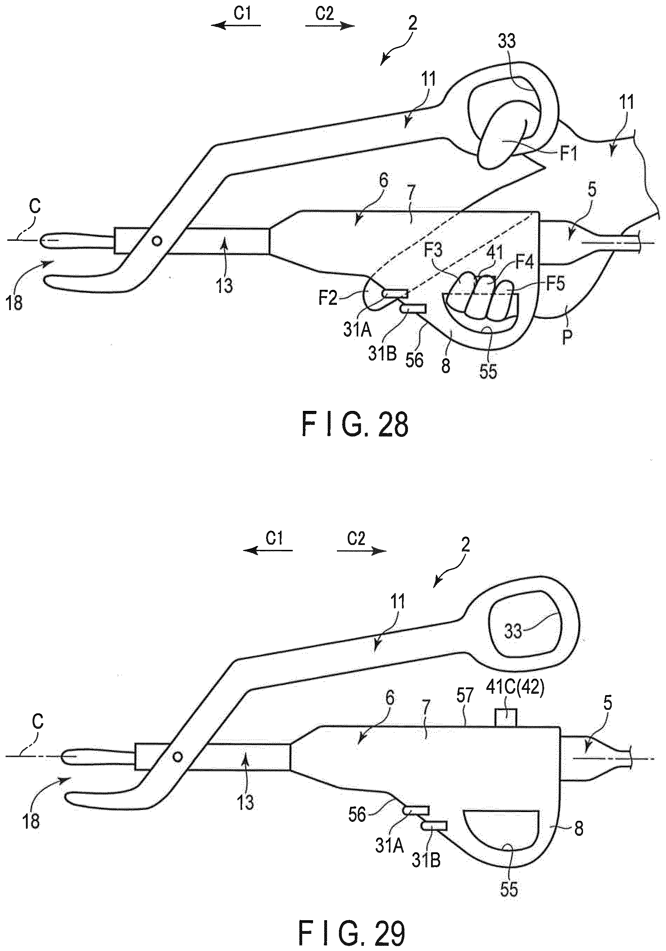

In the embodiments and variations, the handle 11 opens or closes in roughly parallel to the longitudinal axis C. For example, in a thirteenth exemplary variation as illustrated in Figs. 27 and 28, the handle 11 can open or close in a direction crossing (roughly perpendicular to) the longitudinal axis C. In the present exemplary variation, the housing 6 includes a housing body 7 extending along the longitudinal axis C, and a grip 8 extending from the housing body 7 in a direction crossing the longitudinal axis C. The handle 11 is placed opposite to the grip 8 while the longitudinal axis C is centered. The grip 8 includes a grip grasp unit (the grip finger-put position) 55, and an inclined surface 56 placed nearer to the head side of the grip 8 than the grip grasp unit 55. The inclined surface 56 faces the head side of the grip 8. In the present exemplary variation, the operation buttons (the operation members) 31A and 31B are provided on the inclined surface 56 of the grip 8. The operation buttons 31A and 31B are placed nearer to the head side of the grip 8 than the grip grasp unit 55. Thus, the operation buttons 31A and 31B are placed at a portion on the head side of the housing 6.

In the present exemplary variation, the grip grasp unit 55 of the grip 8 is provided with the selection button (the selection member) 41. In the present exemplary variation, the selection button 41 is exposed to the outside of the external surface of the housing 6 and is placed nearer to the base side of the grip 8 than the operation buttons 31A and 31B. In the present exemplary variation, the control unit 26 causes the medical treatment device 1 to function, for example, in one of the patterns X1 to X16, Xa1 to Xa6, Xa9, Xa11 to Xc11, and Xa12 to Xc12 illustrated in Fig. 5 in accordance with the operating state input with the selection button 41.

As illustrated in Fig. 28, for example, in the state in which the right hand H holds the housing 6 and the handle 11, the middle finger F3, ring finger F4, and little finger F5 are put on the grip grasp unit 55 so that the grip 8 of the housing 6 are held. The thumb F1 is put on the force application unit 33 of the handle 11 so that the thumb F1 applies operation force on the handle 11 to close the handle 11 with respect to the grip 8. The index finger F2 performs the operation for rotating the rotating knob 12, or inputting operation with the operation buttons 31A or 31B (pressing operation the operation buttons 31A or 31B) .

In the present exemplary variation, the selection button (the selection member) 41 is placed near to the base side of the housing 6 than the operation buttons 31A and 31B on the external surface of the housing 6, and placed on the grip grasp unit 55. This enables the operator to input operation with the selection button 41 (to press the selection button 41) using any one of the middle finger F3, ring finger F4, and little finger F5 put on the grip grasp unit 55 of the grip 8. The middle finger F3, ring finger F4 and little finger F5 switch the selection button 41 between the ON state and the OFF state. Thus, the thumb F1 and index finger F2 used for at least one of the operations for opening and closing the handle 11, for rotating the rotating knob 12, and for inputting operation with the operation buttons 31A and 31B are not used for inputting operation with the selection button 41. Thus, it is unnecessary to change the positions and postures of the thumb F1 and index finger F2 and to hold the housing 6 and the handle 11 again with the right hand H in order to switch the selection button 41 between the ON state and OFF state in treatment. Thus, in the present exemplary variation, the operability for opening and closing the handle 11, for rotating the rotating knob 12, and for inputting operation with the operation buttons 31A and 31B is secured and the operability for inputting operation with the selection button 41 is also secured.

In a fourteenth exemplary variation illustrated in Fig. 29, the handle 11 opens and closes in a direction crossing (roughly perpendicular to) the longitudinal axis C, and a portion 57 facing the handle 11 on the external surface of the housing 6 is provided with the selection switch 41C (the switch 42). In the present exemplary variation, the selection switch 41C is placed nearer to the base side of the housing 6 than the operation buttons 31A and 31B (the inclined surface 56). The selection switch 41C is exposed to the outside of the external surface of the housing 6. In the present exemplary variation, the selection switch 41C is placed at a position at which the handle 11 can press the selection switch 41C so that opening or closing the handle 11 with respect to the housing 6 varies the pressure from the handle 11 on the selection switch 41C. The variation in the pressure from the handle 11 on the selection switch 41C switches the selection switch 41C between the ON state and the OFF state (in other words, the variation switches the operating states input with the selection switch 41C). In this exemplary variation, the control unit 26 causes the medical treatment device 1 to function, for example, in one of the patterns X1 to X16, Xa1 to Xa6, Xa9, Xa11 to Xc11, and Xa12 to Xc12 illustrated in Fig. 5 in accordance with the operating state on the selection switch 41C.

In the present exemplary variation, the selection switch (the selection member) 41C is placed near to the base side of the housing 6 than the operation buttons 31A and 31B on the housing 6, and placed at a portion 57 facing the handle 11 on the external surface of the housing 6. Opening or closing the handle 11 with respect to the housing 6 can vary the pressure from the handle 11 on the selection switch 41C. In other words, in the present exemplary variation, opening or closing the handle 11 immediately switches the selection switch 41C between the ON state and the OFF state. In the present exemplary variation, the housing 6 and the handle are held as described in the thirteenth exemplary variation (see Fig. 28). Thus, because opening or closing the handle 11 immediately switches the selection switch 41C between the ON state and the OFF state in the present exemplary variation, it is unnecessary to change the positions and postures of the thumb F1 and index finger F2 and to hold the housing 6 and the handle 11 again with the right hand H in order to input operation with the selection switch 41C. Accordingly, in the present exemplary variation, the operability for opening and closing the handle 11, rotating the rotating knob 12, and inputting the operation buttons 31A and 31B is secured and the operability for inputting operation with the selection switch 41C is also secured.

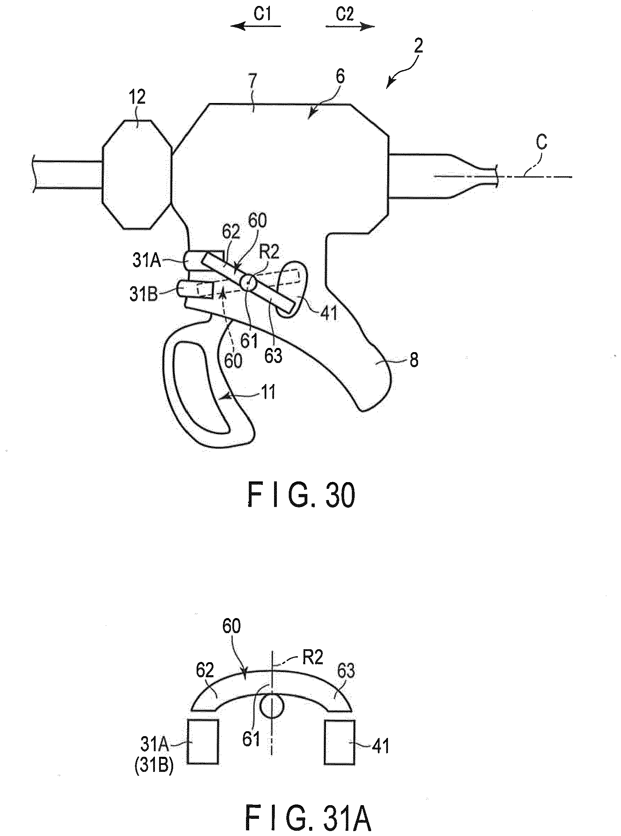

In a fifteenth exemplary variation illustrated in Figs. 30 to 31D, a lever 60 is rotatably attached to the housing 6. Similarly to the first embodiment, the handle 11 is placed nearer to the head side of the housing 6 than the grip 8 in the present exemplary variation. The handle 11 moves in roughly parallel to the longitudinal axis C. Similarly to the first embodiment, the housing 6 is provided with the operation buttons (the operation members) 31A and 31B and the selection button (the selection member) 41. In the present exemplary variation, the lever 60 rotates around a rotation axis R2 that is roughly parallel to the width direction of the housing 6 (the direction perpendicular to the drawing paper of Fig. 30). The lever 60 rotates between a position at which the lever 60 can press the operation button 31A (the position indicated with a solid line in Fig. 30) and a position at which the lever 60 can press the operation button 31B (the position indicated in a dashed line in Fig. 30). Note that the rotation axis R2 of the lever 60 passes through a position nearer to the base side of the housing 6 than the operation buttons 31A and 31B and nearer to the head side of the housing 6 than the selection button 41.

The lever 60 includes a lever supporting shaft 61 through which the rotation axis R2 passes, a first lever extension portion 62 extending from the lever supporting shaft 61 toward the head side of the housing 6 (the side on which the operation buttons 31A and 31B are positioned), and a second lever extension portion 63 extending from the lever supporting shaft 61 toward the base side of the housing 6 (the side on which the selection button 41 is positioned). In order to input operation with the operation button 31A, rotating the lever 60 moves the first lever extension portion 62 to a position at which the first lever extension portion 62 can have contact with the operation button 31A. Meanwhile, the second lever extension portion 63 is positioned at a position at which the second lever extension portion 63 can have contact with the selection button 41. In order to input operation with the operation button 31B, rotating the lever 60 moves the first lever extension portion 62 to a position at which the first lever extension portion 62 can have contact with the operation button 31B. Meanwhile, the second lever extension portion 63 is positioned at a position at which the second lever extension portion 63 can have contact with the selection button 41.

As illustrated in Fig. 31A, even when the lever 60 is positioned at a position at which the lever 60 can have contact with the operation button (31A or 31B) and the selection button 41, the first lever extension portion 62 does not have contact with the operation button (31A or 31B) and the second lever extension portion 63 does not have contact with the selection button 41 unless an external force (the pressing force from the operator) acts on the lever 60. Thus, in the state illustrated in Fig. 31A, operation is not input with the operation button (31A or 31B) and the selection button 41. For example, when the operation button (31A or 31B) and the selection button 41 are momentary ones, the operation button (31A or 31B) and the selection button 41 are in the OFF states.

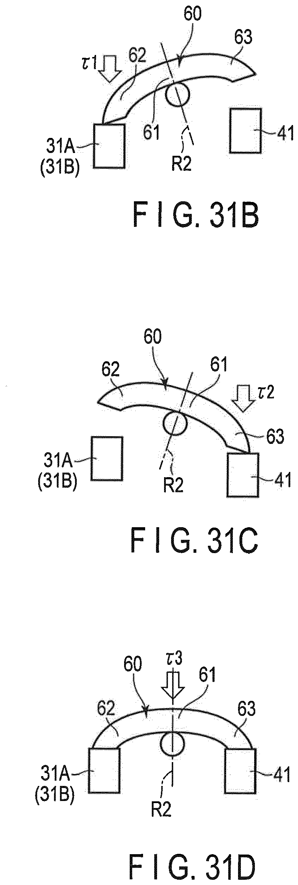

As illustrated in Fig. 31B, when the lever 60 is positioned at a position in which the lever 60 can have contact with the operation button (31A or 31B) and the selection button 41 and an external force (the pressing force from the operator) acts on the first lever extension portion 62 (the arrow τ1 in Fig. 31B), the first lever extension portion 62 has contact with the operation button (31A or 31B). Meanwhile, the second lever extension portion 63 does not have contact with the selection button 41. Thus, in the state illustrated in Fig. 31B, operation is input with the operation button (31A or 31B) while operation is not input with the selection button 41. Thus, for example, when the operation button (31A or 31B) and the selection button 41 are momentary ones, the operation button (31A or 31B) is in the ON state and the selection button 41 is in the OFF state.

On the other hand, when an external force (the pressing force from the operator) acts on the second lever extension portion 63 (the arrow τ2 in Fig. 31C) in a state in which the lever 60 is positioned at a position in which the lever 60 can have contact with the operation button (31A or 31B) and the selection button 41 as illustrated in Fig. 31C, the second lever extension portion 63 has contact with the selection button 41. Meanwhile, the first lever extension portion 62 does not have contact with the operation button (31A or 31B). Thus, in the state as illustrated in Fig. 31C, operation is input with the selection button 41 and operation is not input with the operation button (31A or 31B). Thus, for example, when the operation button (31A or 31B) and the selection button 41 are momentary ones, the selection button 41 is in the ON state and the operation button selection button 41 is in the OFF state.

When an external force (the pressing force from the operator) acts on the lever supporting shaft 61 (the arrow τ3 in Fig. 31D) in a state in which the lever 60 is positioned at a position at which the lever 60 can have contact with the operation button (31A or 31B) and the selection button 41 as illustrated in Fig. 31D, the first lever extension portion 62 has contact with the operation button (31A or 31B) and the second lever extension portion 63 has contact with the selection button 41. Thus, in the state illustrated in Fig. 31D, operation is input with the operation button (31A or 31B) and operation is input with the selection button 31. Thus, for example, when the operation button (31A or 31B) and the selection button 41 are momentary ones, the operation button (31A or 31B) and the selection button 41 are in the ON states.

As described above, in the present exemplary variation, the provision of the lever 60 can makes it easy to simultaneously input operation with both the operation button (31A or 31B) and the selection button 41.

An indicator (not illustrated) that indicates to the operator whether the selection member (41, 41A, 41B, or 41C) is in the ON state or the OFF state can be provided in an exemplary variation. The indicator can be provided on the medical treatment tool 2 (for example, on the housing 6) or on the control device 3. Alternatively, the indicator can be provided separately from the medical treatment tool 2 and the control device 3. The operation of the indicator is controlled, for example, by the control unit 26.

In an embodiment, the indicator is a light generation member such as an LED that generates light when the selection member (41, 41A, 41B, or 41C) in one of the ON state or the OFF state. In another exemplary embodiment, the indicator is, for example, a protrusion or lever provided on the housing. In this example, the switching of the selection member (41, 41A, 41B, or 41C) between the ON state and the OFF state varies the protrusion amount of the protrusion or the orientation of the lever. In other words, the shape of the indicator varies. In another exemplary embodiment, a member that changes, for example, the texture when the selection member (41, 41A, 41B, or 41C) is clicked, the stroke when the selection member (41, 41A, 41B, or 41C) is switched, or the amount of force necessary to switch the selection member (41, 41A, 41B, or 41C) is provided as the indicator. In this example, the switching of the selection member (41, 41A, 41B, or 41C) between the ON state and the OFF state varies the texture, stroke, or the amount of force.

In the embodiments and variations, the medical treatment device (1) is provided with the housing (6) that includes the base and head and includes the housing body (7) extending along the longitudinal axis (C), the handle (11) capable of opening and closing with respect to the housing (6), the end effector (18) placed nearer to the head side of the medical treatment device (1) than the housing (6) and used to treat an object to be treated. A portion on the head side of the housing (6) is provided with the operation member (31A or 31B), a portion nearer to the base side of the housing (6) than the operation member (31A or 31B) is provided with the selection member (41, 41A, 41B, 41C, or 41 and 41C). Operation for causing the medical treatment device (1) to function is input with the operation member (31A or 31B). The operating state input with selection member (41, 41A, 41B, 41C, or 41 and 41C) is switched. The control unit (26) can cause the medical treatment device (1) to function in a plurality of modes and the control unit (26) detects the operating state input with the selection member (41, 41A, 41B, 41C, or 41 and 41C). Then, the control unit (26) selects a mode in which the medical treatment device (1) functions from the modes in accordance with the result of detection of the operating state input with the selection member (41, 41A, 41B, 41C, or 41 and 41C). The configurations of the embodiments and variations can properly be changed or partially combined as long as the configuration includes the components described above.

The embodiments and variations of the present invention have been described above. Needless to say, the present invention is not limited to the embodiments and variations and can variously be changed without departing from the scope of the invention as defined in the appended claims.

## Claims

1. A medical treatment tool (2) used with a control device (3), the control device (3) being configured to control a supply of energy to the medical treatment tool (2), and the control device (3) being capable of functioning in a plurality of operation modes, the medical treatment tool (2) comprising:
a housing (6) which includes a housing body (7) extending along a longitudinal axis (C), and which has a base and a head;
an end effector (18) which is placed nearer to a head side than the housing (6), and which treats an object to be treated;
an operation member (31A, 31B) which is placed at a portion near the head of the housing (6), the operation member (31A, 31B) being configured to be switched between an ON state and an OFF state based on an operation input with the operation member (31A, 31B); and
a selection member (41; 41A; 41B; 41C) which is placed on the housing (6) the selection member (41; 41A; 41B; 41C) being configured to be switched between an ON state and an OFF state based on an operation input with the selection member (41; 41A; 41B; 41C),
**characterized in that**
the control device (3) is configured to function in a first operation mode based on a switching of the operation member (31A, 31B) from the OFF state to the ON state after the control device (3) detects the OFF state of the selection member (41; 41A; 41B; 41C), the first operation mode being one of the plurality of operation modes, and
the control device (3) is configured to function in a second operation mode based on the switching of the operation member (31A, 31B) from the OFF state to the ON state after the control device (3) detects the ON state of the selection member (41; 41A; 41B; 41C), the second operation mode being another one of the plurality of operation modes and being different from the first operation mode.

2. The medical treatment tool (2) according to claim 1, **characterized by** further comprising:
a rotating knob (12) which is placed near the head of the housing (6), and with which operation for rotating the end effector (18) around the longitudinal axis (C) is performed.

3. The medical treatment tool (2) according to claim 1, **characterized in that**:
the operation member (31A, 31B) is arranged at a position at which the operation member (31A, 31B) is operated with an index finger or a middle finger of a hand that holds the housing (6), and
the selection member (41; 41A; 41B) is arranged at a position at which the selection member (41;41A;41B) is operated with a thumb of the hand that holds the housing (6).

4. The medical treatment tool (2) according to claim 1, **characterized in that**:
the selection member (41A; 41B) includes a state maintenance mechanism (48A, 49; 58, 59) which maintains the selection member (41A; 41B) in the ON state or the OFF state.

5. The medical treatment tool (2) according to claim 1, **characterized by** further comprising a handle (11) which is openable and closable with respect to the housing (6),
wherein the selection member (41C) is placed at a position of the housing (6), at which the handle (11) is capable of pressing the selection member (41C), and
wherein the selection member (41C) is switched between the ON state and the OFF state based on variation in pressure from the handle (11) caused by opening or closing operation of the handle (11) with respect to the housing (6).

6. A medical treatment device (2) **characterized by** comprising:
the medical treatment tool (2) according to claim 1; and
the control device (3) which is capable of functioning in the plurality of operation modes.

7. The medical treatment device (1) according to claim 6, **characterized in that**:
the control device (3) supplies a first energy to the end effector (18) by functioning in the first operation, and
the control device (3) supplies a second energy different in kind from the first energy to the end effector (18) by functioning in the second operation mode.

8. The medical treatment device (1) according to claim 6, **characterized in that**:
the control device (3) supplies an energy to the end effector (18) by functioning in the first operation mode, and
the control device (3) supplies an energy that is identical in kind to the energy in the first mode and that is different from the first mode in terms of at least one of an amount of supply, a length of time of supply, and a frequency, by functioning in the second operation mode.

9. The medical treatment device (1) according to claim 6, **characterized by** further comprising a measurement unit (28) which is capable of measuring a predetermined parameter,
wherein the control device (3) supplies an energy to the end effector (18) by functioning in the first operation mode so that the control device (3) causes the end effector (18) to perform treatment using the supplied energy, and
wherein the control device (3) causes the measurement unit (28) to measure the predetermined parameter by functioning in the second operation mode.

10. The medical treatment device (1) according to claim 6, **characterized in that** the control device (3) executes at least one of stopping output of the energy and giving a warning when switching of the selection member (41; 41A; 41B; 41C) between the ON state and the OFF state is detected, until a certain time lapses since the operation member (31A, 31B) is switched from the OFF state to the ON state.

## Patentansprüche

1. Medizinisches Behandlungswerkzeug (2), das mit einer Steuervorrichtung (3) verwendet wird, wobei die Steuervorrichtung (3) konfiguriert ist, um eine Energiezufuhr zu dem medizinischen Behandlungswerkzeug (2) zu steuern, und die Steuervorrichtung (3) in der Lage ist, in einer Mehrzahl von Betriebsmodi zu arbeiten, wobei das medizinische Behandlungswerkzeug (2) umfasst:
ein Gehäuse (6), das einen Gehäusekörper (7) umfasst, der sich entlang einer Längsachse (C) erstreckt, und das eine Basis und einen Kopf aufweist;
einen Endeffektor (18), der näher an einer Kopfseite als das Gehäuse (6) angeordnet ist und ein zu behandelndes Objekt behandelt;
ein Betätigungselement (31A, 31B), das an einem Abschnitt in der Nähe des Kopfes des Gehäuses (6) angeordnet ist, wobei das Betätigungselement (31A, 31B) konfiguriert ist, um basierend auf einer Betätigungseingabe mit dem Betätigungselement (31A, 31B) zwischen einem EIN-Zustand und einem AUS-Zustand umgeschaltet zu werden; und
ein Auswahlelement (41; 41A; 41B; 41C), das an dem Gehäuse (6) angeordnet ist, wobei das Auswahlelement (41; 41A; 41B; 41B; 41C) konfiguriert ist, um basierend auf einer Betätigungseingabe mit dem Auswahlelement (41; 41A; 41B; 41C) zwischen einem EIN-Zustand und einem AUS-Zustand umgeschaltet zu werden,
**dadurch gekennzeichnet, dass**
die Steuervorrichtung (3) konfiguriert ist, um basierend auf einem Umschalten des Betätigungselements (31A, 31B) vom AUS-Zustand in den EIN-Zustand in einem ersten Betriebsmodus zu arbeiten, nachdem die Steuervorrichtung (3) den AUS-Zustand des Auswahlelements (41; 41A; 41B; 41B; 41C) erfasst, wobei der erste Betriebsmodus einer der Mehrzahl von Betriebsmodi ist, und
die Steuervorrichtung (3) konfiguriert ist, um basierend auf dem Umschalten des Betätigungselements (31A, 31B) vom AUS-Zustand in den EIN-Zustand in einem zweiten Betriebsmodus zu arbeiten, nachdem die Steuervorrichtung (3) den EIN-Zustand des Auswahlelements (41; 41A; 41B; 41C) erfasst, wobei der zweite Betriebsmodus ein weiterer der Mehrzahl von Betriebsmodi ist und sich von dem ersten Betriebsmodus unterscheidet.

2. Medizinisches Behandlungswerkzeug (2) nach Anspruch 1, **dadurch gekennzeichnet, dass** es ferner umfasst:
einen Drehknopf (12), der in der Nähe des Gehäusekopfes (6) angeordnet ist und mit dem eine Operation zum Drehen des Endeffektors (18) um die Längsachse (C) durchgeführt wird.

3. Medizinisches Behandlungswerkzeug (2) nach Anspruch 1, **dadurch gekennzeichnet, dass**:
das Betätigungselement (31A, 31B) an einer Position angeordnet ist, an der das Betätigungselement (31A, 31B) mit einem Zeigefinger oder einem Mittelfinger einer Hand bedient wird, die das Gehäuse (6) hält, und
das Auswahlelement (41; 41A; 41B) an einer Position angeordnet ist, an der das Auswahlelement (41; 41A; 41B) mit einem Daumen der Hand bedient wird, die das Gehäuse (6) hält.

4. Medizinisches Behandlungswerkzeug (2) nach Anspruch 1, **dadurch gekennzeichnet, dass**:
das Auswahlelement (41A; 41B) einen Zustandserhaltungsmechanismus (48A, 49; 58, 59) umfasst, der das Auswahlelement (41A; 41B) im EIN-Zustand oder im AUS-Zustand hält.

5. Medizinisches Behandlungswerkzeug (2) nach Anspruch 1, **dadurch gekennzeichnet, dass** es ferner einen Griff (11) umfasst, der in Bezug auf das Gehäuse (6) geöffnet und geschlossen werden kann,
wobei das Auswahlelement (41C) an einer Position des Gehäuses (6) angeordnet ist, an der der Griff (11) das Auswahlelement (41C) drücken kann, und
wobei das Auswahlelement (41C) basierend auf einer Druckänderung an dem Griff (11), die durch eine Öffnungs- oder Schließbetätigung des Griffs (11) in Bezug auf das Gehäuse (6) verursacht wird, zwischen dem EIN-Zustand und dem AUS-Zustand umgeschaltet wird.

6. Medizinische Behandlungsvorrichtung (2), **dadurch gekennzeichnet, dass** sie umfasst:
das medizinische Behandlungswerkzeug (2) nach Anspruch 1; und
die Steuervorrichtung (3), die in der Lage ist, in der Mehrzahl von Betriebsmodi zu arbeiten.

7. Medizinische Behandlungsvorrichtung (1) nach Anspruch 6, **dadurch gekennzeichnet, dass**:
die Steuervorrichtung (3) dem Endeffektor (18) eine erste Energie zuführt, indem sie im ersten Betrieb arbeitet, und
die Steuervorrichtung (3) dem Endeffektor (18) eine zweite Energie zuführt, die sich von der ersten Energie unterscheidet, indem sie in dem zweiten Betriebsmodus arbeitet.

8. Medizinische Behandlungsvorrichtung (1) nach Anspruch 6, **dadurch gekennzeichnet, dass**:
die Steuervorrichtung (3) dem Endeffektor (18) eine Energie zuführt, indem sie in dem ersten Betriebsmodus arbeitet, und
die Steuervorrichtung (3) eine Energie zuführt, die in ihrer Art mit der Energie im ersten Modus identisch ist und sich vom ersten Modus hinsichtlich einer Versorgungsmenge, eine Versorgungsdauer und/oder einer Frequenz unterscheidet, indem sie im zweiten Betriebsmodus arbeitet.

9. Medizinische Behandlungsvorrichtung (1) nach Anspruch 6, **dadurch gekennzeichnet, dass** sie ferner eine Messeinheit (28) umfasst, die in der Lage ist, einen vorbestimmten Parameter zu messen,
wobei die Steuervorrichtung (3) dem Endeffektor (18) eine Energie zuführt, indem sie in dem ersten Betriebsmodus arbeitet, so dass die Steuervorrichtung (3) den Endeffektor (18) veranlasst, eine Behandlung mit der zugeführten Energie durchzuführen, und
wobei die Steuervorrichtung (3) die Messeinheit (28) veranlasst, den vorbestimmten Parameter zu messen, indem sie in dem zweiten Betriebsmodus arbeitet.

10. Medizinische Behandlungsvorrichtung (1) nach Anspruch 6, **dadurch gekennzeichnet, dass** die Steuervorrichtung (3) einen Stopp der Ausgabe der Energie und/oder eine Warnausgabe ausführt, wenn ein Umschalten des Auswahlelements (41; 41A; 41B; 41C) zwischen dem EIN-Zustand und dem AUS-Zustand erfasst wird, bis eine bestimmte Zeit vergeht, seit das Betätigungselement (31A, 31B) vom AUS-Zustand in den EIN-Zustand geschaltet wurde.

## Revendications

1. Outil de traitement médical (2) utilisé avec un dispositif de commande (3), le dispositif de commande (3) étant configuré pour commander une distribution d'énergie à l'outil de traitement médical (2), et le dispositif de commande (3) étant capable de fonctionner dans une pluralité de modes de fonctionnement, l'outil de traitement médical (2) comprenant :
un boîtier (6) qui comprend un corps de boîtier (7) s'étendant le long d'un axe longitudinal (C), et qui a une base et une tête ;
un organe effecteur (18) qui est placé plus près d'un côté tête que le boîtier (6), et qui traite un objet à traiter ;
un élément d'actionnement (31A, 31B) qui est placé à une partie proche de la tête du boîtier (6), l'élément d'actionnement (31A, 31B) étant configuré pour être commuté entre un état MARCHE et un état ARRET sur la base d'une entrée d'opération avec l'élément d'actionnement (31A, 31B) ; et
un élément de sélection (41 ; 41A ; 41B ; 41C) qui est placé sur le boîtier (6), l'élément de sélection (41 ; 41A ; 41B ; 41C) étant configuré pour être commuté entre un état MARCHE et un état ARRET sur la base d'une entrée d'opération avec l'élément de sélection (41 ; 41A ; 41B ; 41C),
**caractérisé par le fait que**
le dispositif de commande (3) est configuré pour fonctionner dans un premier mode de fonctionnement sur la base d'une commutation de l'élément d'actionnement (31A, 31B) de l'état ARRET à l'état MARCHE après que le dispositif de commande (3) détecte l'état ARRET de l'élément de sélection (41 ; 41A ; 41B ; 41C), le premier mode de fonctionnement étant un parmi la pluralité de modes de fonctionnement, et
le dispositif de commande (3) est configuré pour fonctionner dans un second mode de fonctionnement sur la base de la commutation de l'élément d'actionnement (31A, 31B) de l'état ARRET à l'état MARCHE après que le dispositif de commande (3) détecte l'état MARCHE de l'élément de sélection (41 ; 41A ; 41B ; 41C), le second mode de fonctionnement étant un autre parmi la pluralité de modes de fonctionnement et étant différent du premier mode de fonctionnement.

2. Outil de traitement médical (2) selon la revendication 1, **caractérisé par le fait qu'**il comprend en outre :
un bouton rotatif (12) qui est placé près de la tête du boîtier (6), et avec lequel une opération pour faire tourner l'organe effecteur (18) autour de l'axe longitudinal (C) est effectuée.

3. Outil de traitement médical (2) selon la revendication 1, **caractérisé par le fait que** :
l'élément d'actionnement (31A, 31B) est disposé dans une position dans laquelle l'élément d'actionnement (31A, 31B) est actionné avec un index ou un majeur d'une main qui tient le boîtier (6), et
l'élément de sélection (41 ; 41A ; 41B) est disposé dans une position dans laquelle l'élément de sélection (41 ; 41A ; 41B) est actionné avec un pouce de la main qui tient le boîtier (6).

4. Outil de traitement médical (2) selon la revendication 1, **caractérisé par le fait que** :
l'élément de sélection (41A ; 41B) comprend un mécanisme de maintien d'état (48A, 49 ; 58, 59) qui maintient l'élément de sélection (41A ; 41B) dans l'état MARCHE ou l'état ARRET.

5. Outil de traitement médical (2) selon la revendication 1, **caractérisé par le fait qu'**il comprend en outre une poignée (11) qui est apte à s'ouvrir et à se fermer par rapport au boîtier (6),
l'élément de sélection (41C) étant placé dans une position du boîtier (6) dans laquelle la poignée (11) est capable de presser l'élément de sélection (41C), et
l'élément de sélection (41C) étant commuté entre l'état MARCHE et l'état ARRET sur la base d'une variation de pression provenant de la poignée (11) causée par une opération d'ouverture ou de fermeture de la poignée (11) par rapport au boîtier (6).

6. Dispositif de traitement médical (2) **caractérisé par le fait qu'**il comprend :
l'outil de traitement médical (2) selon la revendication 1 ; et
le dispositif de commande (3) qui est capable de fonctionner dans la pluralité de modes de fonctionnement.

7. Dispositif de traitement médical (1) selon la revendication 6, **caractérisé par le fait que** :
le dispositif de commande (3) distribue une première énergie à l'organe effecteur (18) par fonctionnement dans le premier mode de fonctionnement, et
le dispositif de commande (3) distribue une seconde énergie, d'une sorte différente de celle de la première énergie, à l'organe effecteur (18) par fonctionnement dans le second mode de fonctionnement.

8. Dispositif de traitement médical (1) selon la revendication 6, **caractérisé par le fait que** :
le dispositif de commande (3) distribue une énergie à l'organe effecteur (18) par fonctionnement dans le premier mode de fonctionnement, et
le dispositif de commande (3) distribue une énergie qui est de la même sorte que l'énergie dans le premier mode et qui est différente du premier mode en termes d'au moins l'un parmi une quantité de distribution, un laps de temps de distribution et une fréquence, par fonctionnement dans le second mode de fonctionnement.

9. Dispositif de traitement médical (1) selon la revendication 6, **caractérisé par le fait qu'**il comprend en outre une unité de mesure (28) qui est capable de mesurer un paramètre prédéterminé,
le dispositif de commande (3) fournissant une énergie à l'organe effecteur (18) par fonctionnement dans le premier mode de fonctionnement de telle sorte que le dispositif de commande (3) amène l'organe effecteur (18) à effectuer un traitement à l'aide de l'énergie distribuée, et
le dispositif de commande (3) amenant l'unité de mesure (28) à mesurer le paramètre prédéterminé par fonctionnement dans le second mode de fonctionnement.

10. Dispositif de traitement médical (1) selon la revendication 6, **caractérisé par le fait que** le dispositif de commande (3) exécute au moins l'un parmi l'arrêt de l'émission de l'énergie et la fourniture d'un avertissement lorsqu'une commutation de l'élément de sélection (41 ; 41A ; 41B ; 41C) entre l'état MARCHE et l'état ARRET est détectée, jusqu'à ce qu'un certain temps se soit écoulé depuis le moment où l'élément d'actionnement (31A, 31B) est commuté de l'état ARRET à l'état MARCHE.
